# EUROPEAN PATENT APPLICATION

(11) **EP 4 113 535 A1**
(43) Date of publication of application: **04.01.2023**
(21) Application number: 21182428.9
(22) Date of filing: 29.06.2021
(51) Int. Cl.: G16H 50/20

(54) **REMOTE MONITORING METHODS AND SYSTEMS FOR MONITORING PATIENTS SUFFERING FROM CHRONICAL INFLAMMATORY DISEASES**

(71) Applicant: Siemens Healthcare GmbH, 91052 Erlangen (DE)
(72) Inventor: Hanke, Melanie, 90768 Fürth (DE); Jakubcík, Ján, 01001 Zilina (SK); Schaller, Volker, 91052 Erlangen (DE); Vodencarevic, Asmir, 90763 Fürth (DE); Wichmann, Andre, 91054 Buckenhof (DE); Zigo, Peter, 01004 Zilina (SK); Zimmermann-Rittereiser, Marcus, 91091 Grossenseebach (DE)

(57) **Abstract**

The invention relates to a computer-implemented method for determining a predictive disease status of an inflammatory disease of a patient. The method is compatible with a system comprising a remote platform and at least one mobile user device, wherein the user device is associated to the patient and is in data communication with the platform. The method comprises a plurality of steps. One step is directed to receive, at the platform, monitoring data indicative of the health state of the patient from the user device associated to the patient. A further step is directed to determine, at the platform, a predictive disease state of the inflammatory disease of the patient based on the received monitoring data. A further step is directed to evaluate the determined predictive disease status. Yet, a further step is directed to provide the predictive disease status to a user at the platform and/or the patient at the user device based on the step of evaluating the determined predictive disease status. Further, the invention relates to the corresponding system for determining a predictive disease status of an inflammatory disease of a patient.

## Description

The invention relates to methods for enabling a remote monitoring setup for patients suffering from a, in particular chronical, inflammatory disease especially in the home setting of the patients. Further, the invention relates to corresponding systems, computer program products, and computer readable mediums configured to implement such a remote patient monitoring setup.

Inflammatory diseases, such as rheumatic diseases like rheumatoid arthritis, psoriasis arthritis, other musculoskeletal diseases, chronic obstructive pulmonary diseases (COPD), asthma, multiple sclerosis, or Crohn's disease, include a wide range of medical conditions, causing chronic pain and inflammation. For example, rheumatic diseases affect joints, tendons, ligaments, muscles and bones. The most of these conditions occur when the immune system starts attacking its own tissue for often unclear reasons. Often inflammatory diseases are characterized by interlaced periods of disease inactivity, also called "remission", low and moderate disease activity as well as periods of exacerbated (high) disease activity, known as "flares" or "exacerbations".

Most of such diseases are chronic and cannot be cured. To keep the disease activity at low levels, different types of medications are available. It remains challenging for treating physicians and other care personnel, however, to find the right medication and/or the right dosage for an individual patient. Often, an effective treatment needs to be changed to accommodate the patient's current situation (e.g. pausing immunosuppressive therapy due to planned surgery), lower the risk of medication side effects and/or reduce healthcare costs. Furthermore, according to the "treat-to-target" strategy described in the guidelines for treating rheumatoid arthritis, for instance, the dosage of biologic drugs should be tapered once stable remission is achieved. The physician is then again faced with the challenge, which patients are eligible for tapering and how much can the drug be tapered in each individual case. This is often a trial-and-error process, accompanied by reduced patient quality of life and increased healthcare costs until the correct treatment is found.

What is more, suchlike diseases do not take a steady course. Rather, most patients will have to go through repeated cycles of remissions and flares. One issue in this regard is that the treatment these days is mostly reactive. That is, the medication regimes are adapted, and other therapeutical measures are applied, once a flare episode has already started. To improve the quality of life of the patients, it would be desirable to provide means which could predict episodes of flares and/or side effects due to the prescribed medication so that proactive adaptations of the treatment could mitigate the intensity of these events or prevent their occurrence altogether.

One of the problems in this regard is that there is a plethora of individual and external influencing factors that indicate or even trigger the occurrence of adverse event for the individual patient. Individual influencing factors may for instance comprise genetic susceptibility, lifestyle factors like the physical activity, the stress, the diet, the smoking habits, the intake of alcohol and caffeine as well as the vital parameters. External influencing factors are, amongst others, the weather conditions or local environmental conditions such as air pollution values or the concentration of allergens in the air.

Another problem is the lack of availability of information concerning these influencing factors. In the ambulatory clinical routine of rheumatic patients, patients are examined in regular or irregular (in the case of complications like flares) time intervals. During a typical patient visit, which normally lasts 10 to 15 minutes, examination data is collected and sometimes previously collected demographic and lifestyle data is confirmed or updated. Based on this data, the physician makes a treatment decision ideally together with the patient. In between episodes of ambulatory or inpatient treatment, however, there is no possibility to systematically check the wellbeing of the patient to detect early signs of flare or other unwanted events.

It is therefore an object of the present invention to improve existing systems and methods in this regard. In particular, it is an object of the present invention to provide methods and systems that allow for reliable predication of a worsening of the health state of a patient suffering from a, in particular chronical, inflammatory disease in order to, e.g., enable a proactive treatment of an impending flare episode or prevent the occurrence of adverse side effects from the treatment.

This object is solved by methods for determining a predictive clinical condition for an inflammatory disease of a patient and corresponding systems according to one or more of the independent claims. Alternative and/or preferred embodiments are subject of the dependent claims.

In the following, the technical solution according to the present invention is described with respect to the claimed systems as well as with respect to the claimed methods. Features, advantages, or alternative embodiments described herein can likewise be assigned to other claimed objects and vice versa. In other words, claims addressing the inventive method can be improved by features described or claimed with respect to the systems and vice versa. In these cases, functional features of the method may be embodied by objective units, modules, or elements of the system and vice versa.

According to a first aspect, a computer-implemented method for determining a predictive disease status of an inflammatory disease of a patient is provided. The method is compatible with a system comprising a remote platform and at least one mobile user device, wherein the user device is associated to the patient and is in data communication with the platform. The method comprises a plurality of steps. One step is directed to receive, at the platform, monitoring data indicative of the health state of the patient from the user device associated to the patient. A further step is directed to determine, at the platform, a predictive disease state of the inflammatory disease of the patient based on the received monitoring data. A further step is directed to evaluate the determined predictive disease status. Yet, a further step is directed to provide the predictive disease status to a user at the platform and/or the patient at the user device based on the step of evaluating the determined predictive disease status.

In the following, the invention may be described using examples directed to predict the occurrence of flares of rheumatoid arthritis as a predicted disease status. However, the invention is not limited to this application. The invention and its aspects can be used in particular for predicting other disease states or clinical outcomes of a patient having a known inflammatory disease, like e.g. psoriasis arthritis, other musculoskeletal diseases, chronic obstructive pulmonary disease (COPD), asthma, multiple sclerosis, rheumatoid arthritis or Crohn's disease to name only a small number of examples. In particular, inflammatory diseases may be autoimmune diseases where the patient's symptoms may arise from an abnormal immune response to a functioning body part.

According to some examples, the predictive disease status relates to a future status or state of the inflammatory disease of the patient that may occur in the future with a certain probability or likelihood based on the data and information available. As such, the predictive disease status may comprise one or more individual propositions or outcomes relating, e.g., to the wellbeing of the patient, the disease or treatment progression, the side effects of a disease treatment, the occurrence of flares or exacerbations and so forth. In particular, the predictive disease status may be a score for the health state, or a specific condition being monitored such as the occurrence of a flare. According to some examples, the predictive disease status may be or relate to a stage of the inflammatory disease of the patient.

According to some examples, the step of determining a predictive disease status may comprise determining an occurrence probability for a predictive disease status indicating the probability or likelihood that the predictive disease status will occur for the respective patient in a future time period. According to some examples the "future" or the "future time period" may be a predetermined forecast horizon. According to some examples, the forecast horizon may cover a time span of several days up to several weeks to even months from the point in time of the determination of the occurrence probability or the point in time when the monitoring data were taken. In particular, the forecast horizon may be one day to two weeks, preferably one day to five days and more preferably one day to three days. In this regard shorter forecast horizons may lead to higher confidence values for the predictive disease status and/or the occurrence probabilities while longer forecast horizons may provide more time to react, e.g., to account for seasonal changes of the disease activity and for proactively initiating measures countering a potentially negative outcome for the patient, such as sports and increased physical activity as a preventive measure against those seasonal changes. The occurrence probability may relate to a confidence value for the occurrence of a certain disease status in a given forecast horizon.

According to some examples, the remote platform may be a central processing device. The remote platform may comprise a server unit and, in particular, a web server. Further, the remote platform may comprise a cloud server or a local server. The remote platform may include one or more, optionally virtual, computers and/or processing devices and/or processors for processing and analyzing data received from the mobile user devices or other data sources. The remote platform can also include one or more storage devices, such as a mass storage device or a non-volatile memory device for storing data received from the mobile user devices. The remote platform may interface with additional data bases configured to store patient data, e.g., in the form of electronic medical records of patients. The remote platform is generally configured to analyze data received from the mobile user devices and/or the data bases automatically or upon request. The remote platform may include one or more monitoring stations, e.g., for displaying the results of the analysis. For example, healthcare personnel (as the users of the remote platform) can use the one or more monitoring stations to review analysis results generated by the remote platform and/or to receive messages output by the remote platform. The remote platform may be configured to connect and communicate bi-directionally with one or more mobile user devices via an accordingly configured gateway device or interface.

The remote platform may be interfaced with mobile user devices via a network to facilitate data communication between the remote platform and the mobile user devices. The network may comprise the internet and/or a mobile communications network. According to some examples, the data communication between the remote platform and the mobile user devices may be at least partially wireless, e.g., by relying on a Wi-Fi-network and/or a cellular or mobile communications network. The mobile user devices may be configured to connect and communicate bi-directionally with the remote platform via an accordingly configured transceiver device or interface. In some implementations, the remote platform may be configured to interface and/or manage hundreds, thousands, or more mobile user devices related to hundreds, thousands, or more patients. The mobile user devices may generally be configured to collect monitoring data of the patient they are associated to and to forward these data to the remote platform. According to some examples, the mobile user devices may be configured to locally analyze or (pre-)process the collected monitoring data. The mobile user devices may respectively comprise one or more local processors for controlling the collection of monitoring data and, optionally, processing and analyzing the collected monitoring data. To this end, the mobile user devices may run an application downloaded from the remote platform or an App-store, which application is configured to initiate and control the collection and processing of monitoring data at the respective mobile user device. The mobile user devices can also include one or more storage devices, such as a mass storage device or a non-volatile memory device for at least temporarily storing monitoring data.

For collecting monitoring data, the mobile user devices may comprise one or more sensors configured to measure monitoring data and/or may be configured to interface with suchlike sensors. Further, the mobile user device may be configured to access external data sources for collecting monitoring data, e.g., via a network connection such as the internet. The mobile user devices may comprise a user interface for interfacing with the patient and, in particular, receiving input from the patient or outputting messages for the patient as received from the remote platform or as resulting from local data processing at the mobile user device. The term "mobile" may mean that the mobile user devices may be carried and/or worn by the patient. Being associated to the patient may mean that the respective mobile user devices is unambiguously registered with the patient it is associated to. In general, the mobile user devices will respectively be located at a different location than the remote platform, e.g., at the location of the patient, in particular, at the patient's home. According to some examples, the mobile user devices may be smart devices such as but not limited to smart phones, tablet computers or smart watches.

The monitoring data relates to data and values which are currently relevant for the patient's health state. Accordingly, the monitoring data generally indicates a current or at least a recent health state of the patient. According to some examples, the monitoring data may comprise vitals data of the patient, such as the blood pressure, the weight, the blood glucose level, the oxygen saturation level, the body temperature, and the like. Additionally, or alternatively, the monitoring data may comprise information as perceived or inputted by the patient her- or himself such as a perceived wellbeing or the like. Additionally, or alternatively, the monitoring data may comprise environmental data relevant for the patient such as local weather conditions at the location of the patient or the like. In order to gather and transmit the monitoring data, the remote platform may provide a set of instructions to the appropriate mobile user device. For example, the remote platform may provide a set of instructions to a mobile user device to modify operations of a respective set of patient sensors or to query external data bases for relevant information.

Determining a predictive disease status and/or corresponding occurrence probabilities at the remote platform may comprise that the monitoring data is processed and/or that one or more actions related to the monitoring data are performed at the platform (i.e., by the correspondingly configured platform). For example, the remote platform may analyze the monitoring data to determine whether values included in the monitoring data satisfy a threshold (e.g., on a per patient basis), may use a machine learning model to determine whether combinations of values in the vitals data indicate a potential clinical event or issue with the health of a patient, may determine a score for the health of a patient and/or for a specific condition being monitored (e.g., using a machine learning model), and/or the like. In some implementations, the remote platform may process monitoring data/vitals data for hundreds, thousands, or more patients in real-time (or near real-time) .

The step of determining the predictive disease status for a patient may be performed automatically, e.g., once new monitoring data has been received or after a certain time span elapsed after the last predictive health status has been determined. According to some examples, the step of determining the predictive disease status may also be triggered by a user of the remote platform wishing to review a certain patient. Upon triggering the step of determining the predictive disease status, the mobile user devices may automatically be requested by the remote platform to collect and transmit current or new monitoring data.

Evaluating the predictive disease status may, in general, involve assessing whether or not the predictive disease status could potentially entail an unwanted medical condition or outcome for the patient, and, if so, how severe this condition or outcome is. Evaluating the predictive disease status may involve comparing the predictive disease status to a predetermined threshold or rating the predictive disease status according to a medical guideline for the respective inflammatory disease. In particular, evaluating the predictive disease status may comprise comparing corresponding occurrence probabilities to predetermined thresholds. According to some examples, evaluating the predictive disease status may comprise associating a disease stage to the predictive disease status. According to some examples, evaluating the predictive disease status may comprise determining one or more recommendations for the user and/or patient based on the predictive disease status. According to some examples, evaluating the predictive disease status may comprise determining one or more actions for the respective mobile user device and/or the remote platform based on the predictive disease status. The actions may comprise triggering an alert to the user and/or the patient based on the predictive disease status, in particular, if the predictive disease status indicates the occurrence of an unwanted medical condition or outcome for the patient. According to other examples, the actions may comprise initiating an audio and/or video conferencing session between a user at the remote platform and the patient at the mobile user device. According to further examples, the actions may comprise communicating the predictive disease status to a user of the remote platform and/or the patient of the mobile user device, e.g., in the form of a message or indication displayed at the respective user interfaces.

Providing the predictive disease status based on the step of evaluating the predictive disease status may comprise selecting the receiver (i.e., the patient, the user or both), the communication channels for providing the predictive disease status, the priority of the communication of the predictive disease status shall, and so forth. For instance, if the predictive disease state is uncritical for the patient (e.g., if a disease score is below a predetermined threshold), the patient and/or the platform user may just receive a confirmation that everything is fine. If, by contrast, the predictive disease status indicates a potential problem, the remote platform may trigger an alarm, enable a direct communication between patient and platform user, and/or provide further advice to the user and/or the patient. According to some examples, the step of providing may comprise prompting (at the mobile user device and/or the remote platform) the output of a message indicative of the predictive disease status to a user at the platform and/or the patient at the mobile user device.

All in all, these features synergistically work together to improve the monitoring of patients suffering from chronical inflammatory diseases. The usage of mobile user devices to gather monitoring data means that current data about the health state of the patient is readily available even if the patient is in his home setting. With that, a coherent monitoring of patients suffering from chronic inflammatory diseases like rheumatoid arthritis is facilitated. In this regard, the analysis of the monitoring data at a central platform not only ensures that the monitoring data is processed in a controlled environment with sufficient computation power using the latest data analysis tools, but also enables to bring potentially critical conditions to the timely attention of the caregiving personnel. Hence, a provider can proactively react to possible deteriorations of the health state of a patient, e.g., by adjusting the medication or calling the patient in for an ambulatory treatment. This may prevent episodes of suffering for the patient and in turn improve the patient engagement as the patient feels that he is better taken care of.

According to some examples, the predictive disease status comprises a worsening of the disease status, in particular, the occurrence of a "flare" in psoriatic arthritis and rheumatoid arthritis or an "exacerbation" in COPD and asthma. In other words, in the step of determining, the occurrence of a worsening of the disease status of the patient, in particular, a flare or exacerbation, may be predicted. By predicting such episodes and bringing them to the attention of the patient and the healthcare personnel it becomes possible to resort to counter measures before the worsening of the disease status actually occurs.

According to some examples, the predictive disease status comprises a predictive response of the patient to a certain drug (concerning side effects and/or effectiveness). For example, if a patient suffering from rheumatoid arthritis is entering a certain phase of a treatment, the method can estimate, based on current monitoring data, whether this patient will continuously positively respond to drugs that are suggested by corresponding guidelines in that phase. If the method forecasts issues with the patient's drug response, the medication (dose and/or type) can be adjusted early, sparing the patient unnecessary suffering. Further, the method may recognize early signs of unwanted side effects in the monitoring data offering the possibility to proactively counter steer.

According to some examples, the occurrence probability may thus comprise a flare and/or exacerbation probability indicating a probability or likelihood of a flare and/or exacerbation of the inflammatory disease of the patient. According to other examples, the occurrence probability may additionally or alternatively comprise an occurrence probability of side effects related to the treatment of the patient.

According to some examples, the remote platform is in data communication with a local or cloud-based data base for storing electronic medical records of patients. The method further comprises a step of receiving healthcare data associated to the patient from the at least one data base. Further, the method comprises a step of extracting one or more healthcare attributes of the patient from the retrieved healthcare data. Moreover, the step of determining the predictive disease status is additionally based on the extracted one or more healthcare attributes.

In other words, the electronic medical record of the patient may be additionally considered for determining the predictive disease status. This has the advantage that information can be taken into account which cannot be collected by the mobile user devices such as lab data or medical reports from previous examinations of the patient. Accordingly, by combining two disparate data sources - the mobile user device and the electronic medical record of the patient - a holistic image of the health state of the patient can be drawn up. By consequence, the confidence of the predictive disease status may be improved.

The data base may be part of a healthcare information system which is separate to the remote platform. The healthcare information system may comprise one or more databases and servers. The healthcare information system may be a local system within the premises of a healthcare organization. Moreover, the healthcare information system may be cloud based. The healthcare information system may comprise one or more standardized components such as a picture archiving and communication system (PACS), a radiology information system (RIS), a laboratory information system (LIS), a hospital information system (HIS), or an electronic medical record system (EMR). For describing data formats and exchanging electronic health records, corresponding standards may be relied upon such as the HL7 FHIR standard. The remote platform may comprise an interface configured for interfacing with such healthcare information systems and the data bases described herein, in particular, using the HL7 FHIR standard. According to other examples, the data base may also be part of the remote platform, however.

"Receiving" may mean, here, that the healthcare data is retrieved from the healthcare information system. That is, the healthcare data of the patient may be directly retrieved or queried from the data base. Thereby, the resources of the healthcare information system may be queried separately, e.g., on the basis of the patient's ID, name, or other suitable identifier. Moreover, a concerted query may be sent to the healthcare information system which may be configured to manage such data requests internally.

According to some examples, the healthcare data relates to one single patient and preferably comprises data from the group of demographic data (possibly including lifestyle data), medication data, examination data, and lab data. Additionally, the healthcare data may comprise information about an intended change of treatment, e.g., information about a drug intended to be prescribed to the patient or a reduction or increase of a drug dose. Moreover, the healthcare data could potentially include omics data (e.g., proteomics, genomics, metabolomics).

Further, the healthcare data may comprise medical image data acquired using different imaging modalities (e.g., magnetic resonance imaging, ultrasound, computed tomography). In particular, the healthcare data may comprise radiographic image data such as X-Ray image data indicating a radiographic progression of (bone) erosions in arthritic diseases. Further, the healthcare data may also comprise image data acquired with ultrasound or magnetic resonance imaging systems, e.g., indicating a degree of joint swelling.

In addition, or as an alternative, the healthcare data may comprise diffuse optical transmission data. In particular, the diffuse optical transmission data may be acquired by illuminating the hands of the patient with red/near-infrared light and detecting the transmitted light is detected, e.g., by a camera. According to some examples, the diffuse optical transmission data may comprise an optical score indicating the transmissibility/absorption of red/near-infrared light. Since blood is a strong absorber in the red/near-infrared region of the spectrum, pooling of blood will result in a decrease of the transmitted light so that the blood-pooling - which is an indicator for inflammation of tissue - can be quantified.

Moreover, the healthcare data may comprise monitoring data from previous monitoring cycles of the patient. Correspondingly the method may comprise a step of adding the received monitoring data of the patient to the healthcare data of the patient (that is, archiving the monitoring data in the healthcare data of the patient).

The inventors have recognized that all of these data sets are potentially relevant to predict a future disease status of the patient. For instance, the disease progression sensibly depends on the demographic group the patient belongs to and the patient's medical case history.

Accordingly, the one or more healthcare attributes preferably comprise at least one of: a medication prescribed to treat the inflammatory disease of the patient, a medication dose prescribed to treat the inflammatory disease of the patient, demographic information of the patient, laboratory data of the patient, information concerning the patient's lifestyle, and/or examination data of a previous physical examination of the patient. Taking rheumatoid arthritis as an example for an inflammatory disease, the examination data may, for instance comprise the number of swollen joints.

According to some examples, the method further comprises establishing and/or scheduling a bi-directional communication channel between the remote platform and the mobile user device based on the step of evaluating the determined predictive disease status, the communication channel enabling a direct communication between patient and platform user.

For instance, the communication channel may be provided for by a videoconferencing and/or messenger application running at the remote platform and the mobile device. According to other implementations, the communication channel may be based on a telephone connection between the user and the patient.

By enabling a direct communication between the user and the patient, a user my directly get in contact with the patient to gather further information and/or give her/his direct advice to the patient.

According to some examples, the mobile user device is connected to at least one medical information device configured to determine one or more physiological measurement values of the patient, and the monitoring data comprises one or more physiological measurement values as determined by the at least one medical information device.

The physiological measurement values provide an objective measure for the current health state of the patient. Therefore, they provide a good basis for determining a predictive disease status of the patient. Relevant physiological measurement values may, for instance, comprise at least one of:
the patient's blood pressure, the patient's blood glucose levels, the patient's weight and body fat ratio, the patient's physical activity levels, the patient's oxygen saturations levels, the patient's heart rate, the patient's body temperature, and the like. According to some examples, the physiological measurement values may comprise longitudinal data making it possible to extract trends from the data.

Accordingly, the medical information device may preferably be selected from one of: a weight scale, a pulse oximeter, a blood pressure meter, an activity tracker, a blood glucose meter, a thermometer, and the like.

The mobile user device and the medical information devices may be configured such that the physiological measurement values may be automatically transmitted from the medical information devices to mobile user devices. Accordingly, the patient does not have to take care how the measured values are entered into the system. Preferably, the mobile user device and the at least one medical information device are connected wirelessly. In this regard, wireless connection may be effected via a Wi-Fi connection, a Bluetooth connection, a Bluetooth Low Energy (BLE) connection, and/or a ZIGBEE connection, and the like, based on the respective mobile user device being within communicative proximity of the set of medical information devices. In this way, a mobile user device may receive different types of physiological measurement values from different types of medical information devices.

As an alternative or in addition to that, the mobile user device itself may comprise sensors capable of measuring physiological measurement values. For instance, if the mobile user device is a smart watch, the heart rate and other values may be determined directly with the built-in sensors of the watch.

According to some examples, the mobile user device is configured to receive, from the patient, an input indicative of a perceived health state of the patient and the monitoring data comprises the perceived health state.

In other words, the method is configured to record so-called patient-reported outcomes ("PROs"). For instance, the input may comprise the patient's answers to a standardized or customized questionnaire for assessing the health state of the patient. Correspondingly, the method may comprise generating or selecting a questionnaire for the patient (optionally based on the healthcare data and/or the monitoring data and/or the disease type) and providing the questionnaire to patient in order to receive, from the patient responses to the questionnaire as the input.

According to some examples, the questionnaire includes at least one of the Health Assessment Questionnaire (HAQ), the Visual Analog Scale (VAS) questionnaire, and/or the Rheumatoid Arthritis Impact of Disease (RAID) questionnaire. In other words, the input indicative of a perceived health state includes the patient's answers to the Health Assessment Questionnaire (HAQ), the Visual Analog Scale (VAS) questionnaire, and/or the Rheumatoid Arthritis Impact of Disease (RAID) questionnaire. The above questionnaires have proven to be good indicators for future flare episodes.

With that, the subjective impression of the patient regarding his health state may be captured and considered in the ensuing analysis at the remote platform. As this provides an additional layer of information which cannot be captured by measurement values, the quality of the prediction of the disease status can be further improved. In order to allow the patient to input the perceived health state, the mobile user device may run a graphical user interface on a display (which preferably comprises a touch screen) of the mobile user device allowing the user to interact with the system. The corresponding computer program product may be part of the application or app locally running on the mobile user devices and also controlling the other aspects of the data exchange with the platform.

According to some examples, the perceived health state may comprise at least one of: an indication of the patient's perceived wellbeing, a number of swollen joints as determined by the patient, a patient's diet, a medication having been taken by the patient, an occurrence or beginning of a flare or exacerbation event as perceived by the patient, a side effect related to the medication prescribed to the patient as perceived by the patient (like headache, congestion problems, etc.), and the like.

According to some examples, the graphical user interface may be configured such that the patient may input the perceived health state by going through an electronic questionnaire to report various patient-reported outcomes. The questionnaire may, for instance, prompt the patient to rate her or his wellbeing according to a predetermined spectrum (like from one to ten), to input the number of swollen joints, and/or to activate corresponding check boxes if she or he is currently suffering from a potential side effect.

According to some examples, the mobile user device is configured to gather current and/or prospective local environmental information apt to influence the patient's health state, and the monitoring data comprises the local environmental information.

In other words, the mobile user device automatically collects information about external influencing factors which are known to influence the disease status of a patient suffering from an inflammatory disease. For instance, it is known that patients suffering from rheumatoid arthritis react very sensibly to the weather conditions. Accordingly, the local environmental information may comprise at least one of: current weather conditions, current air pollution values, current allergen concentrations, prospective weather conditions, prospective air pollution values, and/or prospective allergen concentrations in the air, and the like. The local environmental information is local in that the corresponding environmental conditions are relevant and may influence the patient at her or his current location - either now or in some predetermined time period in the future.

In order to gather the local environmental information, the mobile user device may rely on built-in sensors which are configured to measure one or more parameters characterizing the local environmental information such as local air quality values. In addition to that or as an alternative, the mobile user device may interface with external sensors configured to provide local environmental information. According to some examples, the mobile user device may also interface with an information network to collect local environmental information. Such information network may for instance be the Internet. Specifically, the mobile user device may use dedicated applications installed on the mobile user device to collect the relevant information from the information network such as commonly known "weather apps". Alternatively, the mobile user device may use a browser application, dedicated communication protocols, or Application Programming Interfaces (APIs) to log on to appropriate web pages providing the corresponding information.

By considering local environmental information, the prediction of a future disease status can be improved. For instance, if it can be determined that the allergen concentration at the location of a COPD patient will be high in the forthcoming week, this may worsen the patient's disease status.

According to some examples, the remote platform is configured to host at least one prediction model configured and/or trained to predict a predictive disease status of an inflammatory disease of a patient based on input data. Further, the step of determining the predictive disease status comprises applying the at least one prediction model to at least the received monitoring data. Input data may be monitoring data and/or healthcare data as herein described. Specifically, the monitoring data may comprise physiological measurement values, patient inputs indicative of a perceived health state of the patient, and/or local environmental information as herein described. Optionally, the one or more prediction models may also be configured to take in healthcare data and extract one or more healthcare attributes of the patient from the healthcare data as herein described and use these to determine the predictive disease status. Accordingly, the step of determining the predictive disease status may comprise also applying the at least one prediction model to the healthcare data.

According to some examples, the remote platform is configured to host a plurality of different prediction models each configured and/or trained to predict a predictive disease status of an inflammatory disease of the patient based on input data. Input data may be monitoring data and/or healthcare data as herein described. The method further comprises a step of selecting one of the hosted prediction models depending on at least the received monitoring data and/or the type of inflammatory disease of the patient and/or the healthcare data. Further, the step of determining the predictive disease status may comprise applying the selected prediction model to at least the received monitoring data and, optionally, to the healthcare data.

The prediction models may be models trained for different purposes. Preferably they have been trained with training data of different groups of patients (each of these models with a training dataset concerning a different group of patients), and/or with training data relating to different medications (although a model may also be trained for a group of different drugs). The models may also be trained with different types of data (e.g., demographic data, medication data, examination data, or lab data), e.g., one model may be trained with laboratory data and one with demographic data. In general, the training of prediction models as well as the architecture of these prediction models are well known in the art (see, e.g., EP 3 573 068 A1).

Each prediction model may be trained to predict the probability of flares, exacerbations, treatment outcomes and so forth based on input data. Treatment outcomes may concern the response of a patient to a drug in a positive way (relief of pain, improvement of the condition), a negative way (side effects) or a neutral way (no response at all). There may be a number of specialized prediction models for different types of inflammatory diseases, different drug-related treatment options, e.g., different doses of a certain group of drugs (or a number of such groups), different drugs, specific patient-groups, different input data and so forth. Preferably, there are in total more than 10 different trained models used, especially more than 30 or even more than 50.

The models may be present in the remote platform itself, such as in a processing unit of the remote platform or in a memory used by the remote platform. For example, information about the architecture and parameters of the prediction models are present in a memory and a chosen prediction model is downloaded from the memory into (a random access memory of) the processing unit.

The selecting step is conceived for automatically selecting one of the available prediction models for the respective use case. Preferably, the prediction model is selected that fits best to the available data, disease type, and treatment scheme and is therefore supposed to provide the best results. The selection may be based on a predefined selection scheme, on data available to be processed by the models, and/or on the respective patient or use case. The selection scheme may be a table stored in a memory of the remote platform or a decision tree hardwired in the computer program product running in the computing unit of the platform.

A prediction model, in general, may be seen as mapping input data to output data thereby fulfilling a certain learned task - here the determination of a predictive disease status of an inflammatory disease of a patient. Prediction models may be based on one or more neural networks (e.g., deep neural networks, recurrent neural networks, convolutional neural networks, convolutional deep neural networks, adversarial networks, deep adversarial networks and/or a generative adversarial networks etc.) and/or other algorithms including Bayesian networks, decision trees, random forest schemes, support vector machines, linear or logistic regression models, gradient averaging, k-means clustering, Q-learning, genetic algorithms and/or association rules or other suitable models and any combination of the aforesaid. Instead of the term "neural network" the term "neuronal net" can also be used. The output generated by the prediction models may depend on one or more parameters of the prediction models. In general, a prediction model may comprise a manifold of parameters. For instance, the parameters may relate to parameters deeply embedded in the prediction model such as the weights in artificial neural networks. Further, the parameters may relate to "superordinate" parameters (often denoted as "hyperparameters") which govern the overall behavior and training of the prediction model. Hyperparameters can be realvalued (e.g., learning rate, width of decision boundaries), integer-valued (e.g., number of layers), binary (e.g., whether to use early stopping or not), or categorical (e.g., choice of optimizer). One example for such hyperparameters would be the parameter "tree depth" in decision trees or random forests which controls the trade-off between achieving a low training error and a low testing error. In addition, the parameters may relate to the type and number of basic trainable algorithms combined to form the prediction model. The one or more parameters of the prediction model can be determined and/or be adjusted by training. The term "prediction model" as used throughout the application relates to prediction models which already underwent training, i.e., the parameters of which have already been determined and/or adjusted by training.

The different types of prediction models which can be hosted in the remote platform may relate to structurally and/or functionally and/or topologically different types or kinds or variants of prediction models. As such, different types may rely on different structures or architectures. For instance, neural networks may comprise a layer structure, while random forest schemes comprise a decision tree structure. Further, the prediction models may fulfill different functions. While some may be configured for feature learning by processing image data, others may be configured to classify data or provide numerical predictions. Further, the prediction models may differ with regard to the learning processes. For instance, one type of prediction models may infer functions from using labeled data pairs by ways of supervised learning. Examples include various kinds of neural networks, decision trees, or Bayesian networks. Other types of prediction models may support unsupervised learning where previously unknown patterns are found in data sets without pre-existing labels or semi-supervised learning. Examples include deep believe nets, hierarchical clustering, or k-means. In terms of machine learning paradigms, yet a third type of prediction models relates to models supporting reinforcement learning which is concerned with how models ought to take actions in an environment so as to maximize some notion of cumulative reward. Examples include Q-learning or learning classifier systems. Furthermore, the prediction models may differ with respect to different metrics optimized during the training process. For instance, for decision tress and tree-based methods in general, these metrics may include information gain, Gini impurity, gain ratio or accuracy.

The determination and/or the adjustment of the one or more parameters of a prediction model during training may occur during the initial creation or compilation of the prediction model from scratch. It may further occur during later training of an already trained prediction model, for instance during re-training or incremental machine learning. Other terms for prediction model may be machine learned model, trained mapping specification, mapping specification with trained parameters, function with trained parameters, algorithm based on artificial intelligence, machine learned algorithm.

Training, in general, may be based on a pair made up of training input data and associated training output data. A trainable prediction model is applied to the training input data for creating model output data. In particular, the determination and/or the adjustment can be based on a comparison of the model output data and the training output data. After training (performance evaluation and deployment), the prediction model may process new unseen data to generate output data according to what it has been trained for. Thus, the training may be seen as implicitly defining the field of application and/or the task the prediction model has to fulfill. Specifically, the training input data and the training output data may be gained from a past disease trajectory of a patient. The training input data may relate to the medical information available for the patient at a certain point in time and comprise monitoring data and healthcare data as herein described. The training output data may be a verified disease status of the patient that actually occurred for the patient sometime after the monitoring data and/or healthcare data comprised in the training input data were recorded. Thereby, "sometime after" may mean within the forecast horizon the prediction model is supposed to work with.

The usage of prediction models in general has the advantage that a more comprehensive and faster screening of the patients connected to the remote platform can be made. In this regard, prediction models may even identify patterns in the available data that are not accessible for a human. Further, the usage of prediction models may enable the swift and at the same time accurate parallel surveillance of a plethora of patients logged on to the remote platform with their user devices. The optional selection of the best prediction model according to the respective patient has the additional advantage of further improved prediction results. It is very complicated (or even impossible) to train one single prediction model for all possible cases and for all possible patients. Furthermore, sometimes it turns out that for a special case (a special group of patients, a special disease, or a special use case), a prediction model of a different architecture is better than a model with another architecture. Thus, by evaluating which prediction model (architecture and training) would be optimal for what case, the accuracy of the prediction can be improved which may foster patient and user engagement.

According to some examples, upon selecting the prediction model, the input data (i.e., monitoring and/or healthcare data) may be searched for predefined data types and/or for values of predefined data types in order to determine whether or not a predefined data type is present in the input data and/or to compare a value with a predefined threshold and/or to decide if the value fits a predefined requirement. For example, it may be determined whether the gender of a patient is male, female or undefined, or whether there is lab-data available in the input data, or if the diagnosis is rheumatoid arthritis. Using this information, an appropriate prediction model may then be selected as a next step.

According to some examples, the method further comprises a step of deriving values of how much individual parameters comprised in the monitoring data and/or the healthcare data affect the predictive disease status, wherein at least one of the derived values and/or the corresponding parameters is provided to a user at the remote platform and/or the patient at the mobile user device.

In other words, information may be output about which input group of parameters affect the output the most. Preferably, a value is generated for individual parameters of this group of how much they affect the output information. This provides a quantitative impression of the importance of these parameters for the patient and/or the user. For example, if it is evident, that for a certain patient a result strongly depends on the body mass index, specific efforts could be made to positively change the body mass index. Moreover, an advantage of this embodiment is that a user could infer from the output that a high flare risk is due to a specific medication regime and selectively change it.

According to some examples, the remote platform is in wireless data communication with a plurality of secondary mobile user devices, each secondary mobile user device being respectively associated to a further patient different from the patient. The method further comprises searching the further patients for similar patients, the similar patients having a degree of similarity to the patient (for which the predictive disease status has been determined and evaluated) and providing the predictive disease status in connection with the similar patients to a user at the remote platform and/or to the similar patients at their respectively corresponding secondary mobile user devices. In particular, "providing" may comprise prompting the output of a message indicative of the predictive disease status in connection with the similar patients to a user at the platform and/or at the respective secondary mobile user devices of the similar patients based on the step of evaluating the determined predictive disease status of the patient. If, for instance, the predictive disease status indicates an increased probability of a flare within the forecast horizon, other patients that are similar to that patient in terms of their heath state and disease history may also have an increased likelihood for a flare in the near future. According to the above example, such risk would automatically be communicated to the similar patients and/or the responsible healthcare personnel at the remote platform to act accordingly.

According to some examples, depending on the predictive disease status, the method may further comprise - in addition to or as an alternative to the previous providing step - requesting secondary monitoring data from the secondary user devices of the similar patients and/or secondary healthcare data for the similar patients from the data base and determining ad-hoc predictive disease states for the similar patients based on the secondary monitoring data and/or the secondary healthcare data. The secondary monitoring data may be of the same or at least similar type and structure as the monitoring data and may comprise similar information and data sets. Likewise, the secondary healthcare data may be of the same or at least similar type and structure as the healthcare data and may comprise similar information and data sets.

With that, it can be verified if the predictive disease status determined for the patient is also pertinent for the similar patients, e.g., by comparing the predictive disease status with the ad-hoc predictive disease states. In particular, this can be done before any further action such as providing the ad-hoc predictive disease, e.g., by way of a communication/message to the users and/or similar patients, is carried out. "Providing" in that respect may depend on the ad-hoc disease states and/or on an evaluation thereof. Accordingly, the similar patient analysis may serve as an additional safety layer for the early identification of medical issues in the monitored patient cohort.

According to some examples, the similarity between the patient and the further patients may be in terms of their respective monitoring data and/or healthcare data. Accordingly, the method may further comprise at least one of the following two steps: receiving, at the platform, secondary monitoring data indicative of the health states of the further patients from the secondary user devices, and/or retrieving secondary healthcare data associated to the further patients from a data base for storing electronic medical records of patients, wherein the remote platform is in data communication with the data base. Further, the step of searching is based on a comparison of the secondary healthcare data with the healthcare data and/or a comparison of the secondary monitoring data with the monitoring data.

Identifying similar patients based on the available monitoring and/or healthcare data is an efficient and accurate way to find patients with similar clinical dispositions that are likely to have similar disease states. According to some examples, it may be preferred to only rely on the healthcare data or secondary healthcare data in this regard as this data may be more readily available and usually already provides sufficient information to at least identify "candidate" similar patients. Whether or not the predictive disease status really can be transferred to these candidate similar patients can then, in a next step, be scrutinized by requesting and analyzing the secondary monitoring data of these patients as herein described elsewhere.

According to some examples, the step of comparing may comprise extracting a data descriptor from the monitoring and/or healthcare data and extracting a corresponding data descriptor from each of the secondary healthcare and/or monitoring data sets, with the degree of similarity between the patient and the further patients then respectively being based on a similarity between the data descriptor and individual corresponding data descriptors. In particular, similar patients may be those further patients, the degree of similarity of which is higher than a predetermined threshold.

In some examples, a data descriptor may be a vector representing or characterizing the healthcare and/or monitoring data in a particular vector space. In some examples, a data descriptor may be determined by encoding the healthcare and/or monitoring data using a particular data descriptor encoding algorithm. Features of the healthcare and/or monitoring data, such as vital parameters, demographic information, the disease type and trajectory, information about the medication, lifestyle, and so forth are represented or characterized by the data descriptor.

According to some examples, the degrees of similarity may be calculated by applying a similarity metric representing how similar the respective healthcare and/or monitoring data sets are. In some examples, the similarity metric may be a distance in vector space between the data descriptor of the healthcare and/or monitoring data and the respective data descriptors of the secondary healthcare and/or monitoring data sets. In other examples, further similarity metrics may be used, such as a cosine similarity between the data descriptors. According to some embodiments, the calculation of the data descriptors and/or the identification of similar patients as a whole may be performed by one or more computerlearned functions.

According to some examples, the method may further comprise a step of providing a predetermined association, the predetermined association linking different stages of predictive disease status to actions and/or recommendations for the patient and/or a user of the platform. Further, the step of evaluating may comprise identifying a stage of the determined predictive disease status and selecting actions and/or recommendations based on the identified stage and the predetermined association. Moreover, the step of providing comprises providing the selected actions and/or recommendations to a user at the remote platform and/or the patient at the mobile user device.

Many inflammatory diseases are ranked according to different stages. For COPD, for instance, there are commonly four stages reaching from "early" to "very severe". Each stage foresees different recommendations for the patient but also entails different treatment options for the healthcare personnel. Determining the stage of the predictive disease status thus enables to automatically identify the best recommendations for the patient and/or the healthcare personnel. Depending on the stage, a recommendation for the patient could reach from merely "increase your physical activity level" to "seek medical assistance immediately".

According to some examples, a prediction model may be provided to the mobile user device which prediction model is configured and/or trained to locally determine, at the mobile user device, a predictive disease status of the patient based on the monitoring data.

By providing a prediction model to the mobile user device, data can be processed locally, which may be beneficial in that less data needs to be transmitted to the platform. Further, the patient may be provided with means to locally assess his health state even in times where he cannot connect to the remote platform. Besides generating a prediction of a future disease status and/or corresponding occurrence probabilities, the results locally provided by the prediction models at the user devices may be used for improving the underlying prediction model for an induvial patient in an incremental and/or self-learning manner. In addition, or as an alternative, the results may be used for improving predictions and/or corresponding occurrence probabilities of other patients if sent send to the remote platform for further processing. At the remote platform these results may then be used to adapt the existing models at the remote platform in a federated learning fashion.

According to further examples, the mobile user devices may further be configured to host a plurality of different prediction models which can be selected depending on the respective circumstance essentially as described before.

According to some examples, the method may further comprise receiving, at the platform, a locally determined predictive disease status and comparing, at the platform, the locally determined predictive disease status for the patient with the predictive disease status determined at the remote platform. With that, a safety check can be performed in order to determine whether or not the prediction models at the platform and/or the mobile user devices are still working properly.

According to some examples, the method may further comprise receiving, at the platform, a locally determined predictive disease status. Depending on the locally determined predictive disease status the method may further comprise requesting (and receiving) monitoring data and or healthcare data for the patient and determining a predictive disease status at the remote platform based on the monitoring and/or healthcare data. Accordingly, the locally determined disease status may be evaluated and/or checked at the remote platform. If the locally determined disease status indicates a potential health issue of the patient, this may be verified by determining a predictive disease status at the platform with all the information available. In other words, the locally determined disease status may be used as an indicator whether or not a more detailed analysis is in order. With that, the monitoring of the patients can be further improved without necessarily having to increase the frequency at which predictive disease states are to be calculated at the platform.

According to some examples, the method further comprises further training of one or more prediction models based on the healthcare data, the monitoring data and/or one or more predictive disease states.

Besides determining a predictive disease status, either at the remote platform or on the mobile device, the available data is thus being used for further improving one or more prediction models. For instance, it is conceivable to retroactively verify a (prior) predictive disease status based on (newly gathered) monitoring data. Correspondingly, the method may comprise receiving a prior predictive disease status predicted by a prediction model and further training the prediction model based on the prior predictive disease status and the monitoring data and/or the healthcare data. According to some examples, the prior predictive disease status may be received from the healthcare data (it previously has been added to) .

According to some examples, the further training may be triggered automatically in a self-learning fashion. According to further examples, the further training may involve incremental learning essentially as described in EP 3 798 934 A1.

The steps according to the above aspect(s) preferably happen "platform-side", i.e., at the remote platform, or are at least initiated by the remote platform. They may be complemented by corresponding "user device-side" steps happening at the mobile user devices. Such steps may comprise at least one of: collecting monitoring data indicative of the health state of the patient, transmitting the monitoring data to the remote platform, preferably in order to induce, at the platform a determination of a predictive disease status for the patient based on the transmitted monitoring data, receiving, from the platform, information indicative of the determined predictive disease status, outputting, to the patient, a message indicative of the received information, receiving a prediction model configured and/or trained to locally determine, at the mobile user device, a predictive disease status of the patient based on the monitoring data, determine a predictive disease status for the patient by locally applying the prediction model to the monitoring data, outputting, to the patient, a message indicative of the locally determined predictive disease status, and/or transmitting the locally determined predictive disease status to the platform.

According to a further aspect, a computer-implemented method for determining a predictive disease status for an inflammatory disease of a patient is provided, in a system comprising a remote platform and at least one mobile user device, the mobile user device being associated to the patient and being in wireless data communication with the platform. The method comprises a plurality of steps. One step is directed to collect, at the mobile user device, monitoring data indicative of the health state of the patient. A further step is directed to transmit the monitoring data to the remote platform, preferably in order to induce, at the remote platform a determination of a predictive disease status of the inflammatory disease for the patient based on the transmitted monitoring data. A further step is directed to receive, at the mobile user device from the remote platform, information indicative of the predictive disease status. A further step is directed to output, to the patient, a message indicative of the received information.

Advantages and modifications disclosed in connection with the foregoing aspect and examples can likewise be realized by or may be applied to the above method.

According to some examples, the method may further comprise the steps of: receiving, at the mobile user device, a prediction model configured and/or trained to locally determine, at the mobile user device, a predictive disease status of the patient based on the monitoring data, locally determine a predictive disease status for the patient by locally applying the prediction model to the monitoring data, and outputting, to the patient, a message indicative of the locally determined predictive disease status and/or transmitting the locally determined predictive disease status to the remote platform.

Algorithms on mobile devices may also train mathematical models, that underly the determination of predictive disease states, locally on their mobile device, and subsequently transmit those trained models to the remote platform, at which the models may be combined with other models, which have been trained on other mobile devices, resulting in models, that further increase the confidence of predictions of future disease states and/or of corresponding occurrence probabilities for the ensemble of patients.

According to a further aspect, a patient monitoring platform for determining a predictive disease status for an inflammatory disease of a patient is provided. The platform comprises an interface and a computing unit. The interface is configured to communicate with at least one mobile user device being associated to the patient for receiving monitoring data indicative of the health state of the patient from the mobile user device. The computing unit is configured to determine a predictive disease status for the inflammatory disease of the patient based on the received monitoring data, evaluate the determined predictive disease status, and provide the predictive disease status based on the evaluation, in particular, to a user at the platform, e.g., by outputting a message indicative of the predictive disease status to a user at the platform, or to the patient, e.g., by prompting the mobile user device via the interface unit to output a message indicative of the predictive disease status to the patient.

According to an embodiment, the platform is adapted to implement the inventive methods of providing a predictive disease status of a patient suffering from an inflammatory disease according to the aspects and examples as herein disclosed.

The computing unit may be realized as a data processing system or as a part of a data processing system. Such a data processing system can, for example, comprise a cloudcomputing system, a computer network, a computer, a tablet computer, a smartphone and/or the like. The computing unit can comprise hardware and/or software. The hardware can comprise, for example, one or more processors, one or more memories and combinations thereof. The one or more memories may store instructions for carrying out the method steps according to the aspects and examples as herein disclosed. The hardware can be configurable by the software and/or be operable by the software. Generally, all units, sub-units or modules may at least temporarily be in data exchange with each other, e.g., via a network connection or respective interfaces. Consequently, individual units may be located apart from each other.

The interface unit may comprise an interface for data exchange with one or more mobile user devices via internet connection. Further, the interface unit may comprise an interface for data exchange with a data base storing healthcare data of one or more patients. The interface unit may be further adapted to interface with one or more users of the remote platform (i.e., the responsible healthcare personnel) via a user interface, e.g., by displaying the predictive disease status to the user (e.g., in a graphical user interface) .

According to some examples, the platform may further comprise a data base for storing healthcare data of a plurality of patients. The data base may be configured according to the aspects and examples as herein described.

According to an aspect, a system for providing a predictive disease status for an inflammatory disease of a patient is provided. The system comprises the remote platform according to the previous aspects and examples and at least one mobile user device associated to the patient, the mobile user device being configured to collect monitoring data indicative of the health state of the patient and to transmit the monitoring data to the platform. Specifically, the mobile user device may comprise a mobile computing unit and a mobile interface unit. The mobile computing unit can comprise hardware and/or software. The hardware can comprise, for example, one or more processors, one or more memories and combinations thereof. The one or more memories may store instructions for carrying out method steps according to aspects and examples as herein described. The hardware can be configurable by the software and/or be operable by the software. In particular, the computing unit may comprise a processor of a portable user device such as a smart phone or smart watch or tablet computer. Further, the mobile computing unit may comprise an integrated circuit that integrates all or most components of the mobile user device (system on a chip). The mobile interface unit may comprise an interface for, preferably at least partial wireless, data exchange with the remote platform, e.g., via an internet connection. Further, the mobile interface unit may comprise an interface for, preferably at least partial wireless, data exchange with one or more medical information devices, e.g., using the Bluetooth standard. The mobile interface unit may be further adapted to interface with the patient via a user interface, e.g., by displaying the predictive disease status to the patient (e.g., in a graphical user interface) and/or by allowing the patient to input information about her or his perceived health state.

According to another aspect, the present invention is directed to a computer program product comprising program elements which induce a computing unit of a remote platform and/or a mobile user device and/or a system comprising the remote platform and one or more mobile user devices to perform the steps according to the methods as herein described, when the program elements are loaded into memories of the respective computing units.

According to another aspect, the present invention is directed to a computer-readable medium on which program elements are stored that are readable and executable by a computing unit of a remote platform and/or a mobile user device and/or a system comprising the remote platform and one or more mobile user devices, in order to perform steps according to the methods as herein described, when the program elements are executed by the respective computing unit.

The realization of the invention by a computer program product and/or a computer-readable medium has the advantage that already existing providing systems can be easily adapted by software updates in order to work as proposed by the invention.

The computer program product can be, for example, a computer program or comprise another element next to the computer program as such. This other element can be hardware, e.g., a memory device, on which the computer program is stored, a hardware key for using the computer program and the like, and/or software, e.g., a documentation or a software key for using the computer program. The computer program product may further comprise development material, a runtime system and/or databases or libraries. The computer program product may be distributed among several computer instances.

### Brief Description of the Drawings

Characteristics, features and advantages of the above described invention, as well as the manner they are achieved, become clearer and more understandable in the light of the following description and embodiments, which will be described in detail with respect to the figures. This following description does not limit the invention on the contained embodiments. Same components, parts or steps can be labeled with the same reference signs in different figures. In general, the figures are not drawn to scale. In the following:
FIG. 1 depicts a system for remote monitoring of a patient suffering from an inflammatory disease according to an embodiment;
FIG. 2 depicts a remote platform for remote monitoring of a patient suffering from an inflammatory disease according to an embodiment;
FIG. 3 depicts a mobile user device for remote monitoring of a patient suffering from an inflammatory disease according to an embodiment;
FIG. 4 depicts a flowchart illustrating a method for providing a predictive disease status of a patient according to an embodiment;
FIG. 5 depicts a flowchart illustrating a method for providing a predictive disease status of a patient according to a further embodiment;
FIG. 6 depicts a flowchart illustrating a method for providing a predictive disease status of a patient according to a further embodiment;
FIG. 7 depicts a flowchart illustrating a method for providing a predictive disease status of a patient according to a further embodiment;
FIG. 8 depicts a flowchart illustrating a method for providing a predictive disease status of a patient according to a further embodiment;
FIG. 9 depicts a flowchart illustrating a method for providing a predictive disease status of a patient according to a further embodiment; and
FIG. 10 depicts a flowchart illustrating a method for providing a predictive disease status of a patient according to a further embodiment.

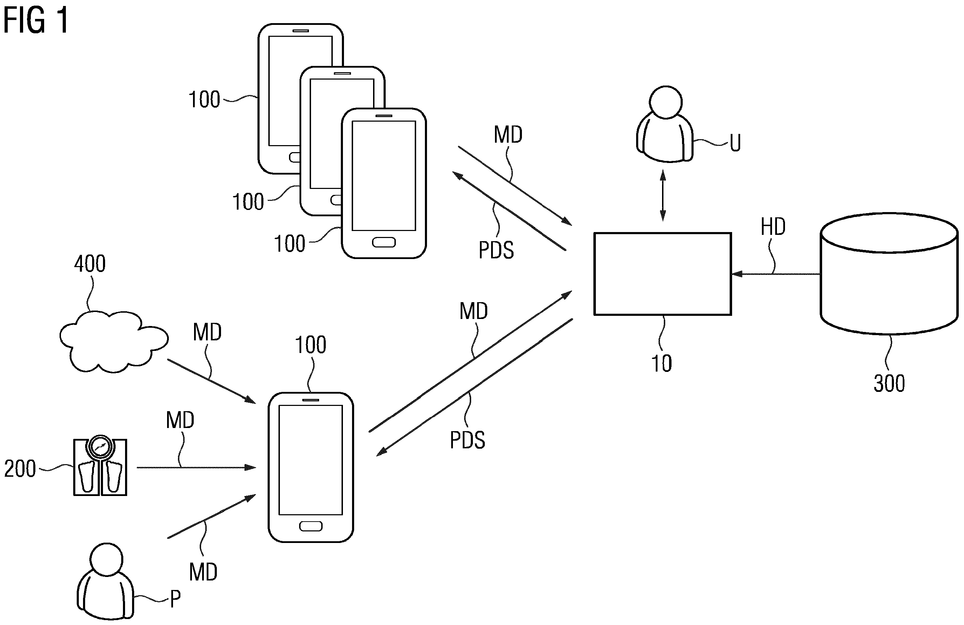

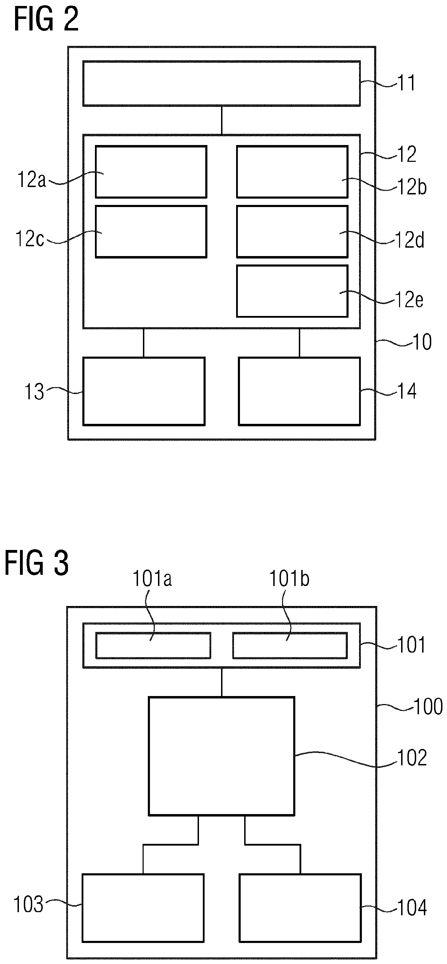

**Figure 1** schematically shows a system 1 for remote monitoring a patient P suffering from an inflammatory disease according to an exemplary embodiment. **Figure 2** schematically shows a remote platform 10 of system 1 according to an embodiment.

**Figure 3** schematically shows a mobile user device 100 of system 1 according to an embodiment.

As shown in Figure 1, system 1 includes at least one, preferably a plurality of mobile user devices 100. The mobile user devices 100 are respectively associated to a patient P being enrolled in the patient monitoring program as provided by system 1. The mobile user devices 100 are in data exchange with a remote platform 10 which provides capabilities for analyzing monitoring data MD as collected by the respective mobile user devices 100. To this end, the mobile user devices 100 each may be in data communication with a medical information device 200 configured to measure monitoring data MD (directly) at patient P and/or in the home setting of patient P. Further, mobile user devices 100 may be configured to collected monitoring data MD from external data sources 400 such as the internet and/or infer monitoring data from direct inputs of patient P into mobile user devices 100. Remote platform 10 is furthermore in data exchange with a data base 300 storing healthcare data HD of at least a part of the patients P connected to the remote platform 10 via their individual mobile user devices 100.

In general, system 1 is capable of determining a predictive disease status of a patient P suffering from an inflammatory disease based on locally gathered monitoring data MD and/or healthcare data HD taken from the respective patient's electronic medical records.

Monitoring data MD may relate to data and values which characterize the respective patient's current or recent health state. According to some examples, monitoring data MD may comprise vitals data of the patient, such as the blood pressure, the weight, the blood glucose level, the oxygen saturation level, the body temperature and the like as locally measured by one or more medical information devices 200. Additionally or alternatively, monitoring data MD may comprise information inputted into mobile user device 100 by patient P, such as various PROs collected via digital symptom questionnaires. Additionally, or alternatively, monitoring data MD may comprise local environmental data relevant for respective patient P such as local weather conditions or the like.

Healthcare data HD generally relates to data from the electronic medical records of the patients P. As such, healthcare data HD optimally complement the information from the monitoring data MD as the healthcare data HD provide further background information to optimally judge the patient's health state and predict a future disease status. Healthcare data HD preferably comprises data from the group of demographic data (possibly including lifestyle data), medication data, examination data and/or lab data. Additionally, healthcare data HD may comprise information about an intended change of treatment, e.g., information about a drug intended to be applied to the patient or a reduction or increase of a drug dose. Moreover, healthcare data HD may potentially inelude omics data (e.g., proteomics, genomics, metabolomics) and medical image data acquired using different imaging modalities (e.g., magnetic resonance imaging, ultrasound, computed tomography). Healthcare data HD may also comprise one or more medical reports of past examinations of the patient P. Moreover, healthcare data HD may comprise monitoring data from previous monitoring cycles which have been added to the healthcare data by remote platform 10. In other words, remote platform may be configured to push monitoring data MD to data base 300 (or the corresponding healthcare information system) .

Medical information devices 200 may generally be configured to measure, generate, store, (pre-)process, and/or provide monitoring data MD of patient P. In particular, medical information devices 200 may be configured to provide physiological measurement values acquired from patient P or in relation to patient P.

Medical information devices 200 each may include one or more sensor devices capable of measuring, generating, and/or providing physiological measurement values of patient P. For example, such sensor devices may include a blood pressure monitor, an oximeter, a glucometer, an airflow sensor, a galvanic skin response sensor, an electrocardiogram sensor, a heart rate monitor, a scale, a pulse oximeter, a thermometer, or a similar type of device. In some implementations, such sensor devices may be implemented within a wearable device or even mobile user device 100 itself (e.g., a smart wristwatch, a pair of smart eyeglasses, an activity band, and/or the like). In some implementations, medical information devices 200 may provide monitoring data MD to mobile user devices 100 after gathering monitoring data MD. Additionally, or alternatively, medical information devices 200 may also be controlled remotely by remote platform 10 and interface directly with remote platform 10.

According to some examples, a patient P may be associated with multiple medical information devices 200 that are of different types and/or manufactured by different third parties. For example, a patient P may be associated with a blood pressure monitor and a heart rate monitor, which are gathering monitoring data MD of patient P. Further medical information devices 200 may also include sensor devices configured to gather local environmental data of an environment in which patient P is located (e.g., air humidity values, air temperature values, whether particular chemicals are present in the environment, the concentration of allergens in the air and/or the like).

Mobile user device 100 generally is a device configured to receive, generate, store, process, and/or provide monitoring data MD related to a patient P. For example, mobile user device 100 may include a mobile phone (e.g., a smart phone, a radiotelephone, etc.), a laptop computer, a tablet computer, a handheld computer, a gaming device, or a wearable communication device (e.g., a smart wristwatch, a pair of smart eyeglasses, etc.).

In some implementations, mobile user device 100 may function as a gateway device between medical information devices 200 and remote platform 10. That is, mobile user device 100 may function as a communication intermediary between medical information devices 200 and remote platform 10.

As shown in Figure 3, mobile user device 100 comprises a computing unit 102 (also denoted as mobile computing unit). Computing unit 102 may be embodied by one or more built-in processors of mobile user device 100 and/or may be implemented in hardware, software, or a combination of hardware and software. Computing unit 102 may be a central processing unit (CPU), a graphics processing unit (GPU), an accelerated processing unit (APU), a microprocessor, a microcontroller, a digital signal processor (DSP), a field-programmable gate array (FPGA), an application-specific integrated circuit (ASIC), a Neural Processing Unit (NPU), a system on a chip device or another type of processing component. In some implementations, computing unit 102 includes one or more processors capable of being programmed to perform one or more method steps as herein described.

As shown in Figure 3 mobile user device 100 may include an interface unit 101 (also denoted as mobile interface unit) for facilitating communication with platform 10 and one or more medical information devices 200. In general, interface unit 101 may include a transceiver-like component (e.g., a transceiver and/or a separate receiver and transmitter) that enables mobile user device 100 to communicate with other devices, such as via a wired connection, a wireless connection, or a combination of wired and wireless connections. Interface unit 101 may permit mobile user device 100 to receive information from another device and/or provide information to another device. For example, interface unit 101 may include an Ethernet interface, an optical interface, a coaxial interface, an infrared interface, a radio frequency (RF) interface, a universal serial bus (USB) interface, a Wi-Fi interface, a cellular network interface, or the like.

According to some examples, interface unit 101 may include a sensor interface 101a which may provide mobile user device 100 with the capabilities needed to communicate with respective medical information devices 200. For instance, sensor interface 101a may provide mobile user device 100 with a capability to use a protocol associated with a particular medical information device 200, e.g., to use particular commands associated with that medical information device 200, and so forth. In some implementations, sensor interface 101a may communicate to discover a medical information device 200, to start and/or pause data collection by a medical information device 200, and/or the like. In some implementations, sensor interface 101a may include one or more wireless adapters, e.g., in the form of a Bluetooth, Wi-Fi, or ZIGBEE adapter. Such wireless adapters may provide mobile user device 100 with a capability to perform bi-directional data communication with medical information devices 200, such as initiating device discovery, querying a list of paired devices, creating a server socket to listen for connection requests, and/or the like. In some implementations, sensor interface 101a may communicate with corresponding wireless adapters at medical information devices 200 so that communications (e.g., instructions, physiological measurement values, etc.) can be exchanged between mobile user device 100 and one of the medical information devices 200.

In addition, interface unit 101 may comprise a network interface 101b to communicate with remote platform 10 and/or to access external information sources via the internet. For example, network interface 101b may provide mobile client device 100 with a capability to communicate with remote platform 10 via an HTTP or HTTPS connection or to access web pages and/or web services provided via the internet. According to some examples, network interface 101b is implemented as a wireless interface based on a Wi-Fi or WLAN connection. According to other examples, network interface 101b is implemented as a (wireless) interface based on cellular network (e.g., a long-term evolution (LTE) network, a code division multiple access (CDMA) network, a 3G network, a 4G network, a 5G network, another type of next generation network, etc.), a public land mobile network (PLMN), a local area network (LAN), a wide area network (WAN), a telephone network (e.g., the Public Switched Telephone Network (PSTN )), a private network, an ad hoc network, an intranet, the Internet, a fiber optic-based network, a cloud computing network connection and/or a combination of these or other types of network connections.

As shown in Figure 3, mobile user device 100 may include a user interface 103, which according to some implementations may be part of interface unit 101. User interface 103 may be configured to interface with patient P in order to display information and/or control mobile user device 100. To this end, computing unit 102 may run dedicated applications related to displaying information and controlling mobile user device 100. As a specific example, computing unit 102 may run a gateway application that is associated with displaying physiological measurement values and/or that communicates with sensor interface 101a to control data capture from one of the medical information devices 200. User interface 103 may comprise devices for user input (e.g., a touch screen display, one or more buttons, a switch, and/or a microphone). Additionally, user interface 103 may comprise devices that provide output information from mobile user device 100 (e.g., a display, a speaker, and/or one or more light-emitting diodes) .

In some implementations, mobile user device 100 may include a memory 104 (also denoted as mobile memory) which may receive and/or store physiological measurement values from medical information devices 200 or other monitoring data MD. In particular, memory 104 may serve as an offline data buffer for storing monitoring data MD for later transmission to remote platform 10 when mobile user device 100 is not connected to remote platform 10. Further, memory 104 may store information and/or software related to the operation and use of mobile user device 100. Memory 104 may include a random access memory (RAM), a read only memory (ROM), a hard disk drive, a solid state drive and/or another type of dynamic or static storage device (e.g., a flash memory, a magnetic memory, and/or an optical memory).

Remote platform 10 is generally configured to request, receive, store, process, monitoring data MD and healthcare data HD related to a patient P in order to determine a predictive disease status of the inflammatory disease of patient P.

As shown in Figure 2, remote platform 10 in the depicted implementation comprises an interface unit 11, a computing unit 12, a user interface 13 and a memory unit 14. According to some examples, remote platform 10 may be hosted in cloud computing environment. Noteworthy, while examples described herein describe remote platform 10 as being hosted in a cloud computing environment, remote platform 10 may also not be cloud-based (i.e., may be implemented outside of a cloud computing environment) or may be partially cloud-based according to other examples. The cloud computing environment may provide computation, software, data access, storage, and/or other services that do not require end-user knowledge of a physical location and configuration of a system and/or a device that hosts remote platform 10.

Computing unit 12 may include a group of computing resources which may include one or more personal computers, workstation computers, server devices, or another type of computation and/or communication device. According to some examples, computing unit 12 may communicate with other computing resources via wired connections, wireless connections, or a combination of wired and wireless connections. Further, computing unit 12 may include a group of cloud resources, such as one or more applications or one or more virtual machines. The applications may include one or more software applications that may be provided to or accessed by one or more devices of system 1. According to some examples, the applications may include a software application associated with one or more data bases 300.

Computing unit 12 may comprise a data retrieval module 12a configured to retrieve healthcare data HD from data base 300 and/or monitoring data MD from the connected mobile user devices 100. Further, computing unit 12 may comprise an analysis or prediction module 12b configured to determine a predictive disease status PDS of a patient P based on monitoring data MD and/or healthcare data HD retrieved for that patient P by retrieval module 12a. According to some examples, prediction module 12b may be configured to host one or more prediction models configured and trained for this task and to apply one or more prediction models to monitoring data MD and/or healthcare data HD. To select an appropriate prediction model from a plurality of available prediction models, computing unit 12 may optionally comprise a selection module 12c. Selection module 12c may be configured to select, from a plurality of prediction models hosted at the prediction module 12b, one prediction model which is suited best for the respective monitoring data MD and/or healthcare data HD or, generally, the respective patient case. Optionally, computing unit 12 may also comprise a similar patient search module or SPS module 12d. SPS module 12d may be configured to search, amongst the patients enrolled with the monitoring program, for patients that are somewhat similar to patient P currently investigated (i.e., the patient P for which the predictive disease status PDS has been determined). Moreover, computing unit 12 may comprise a communication module 12e for initiating communication with the patients P at mobile user devices 100 and/or users U (i.e., healthcare personnel) at the remote platform 10. The designation of the distinct modules 12a-12e is to be construed by way of example and not as limitation. Specifically, modules 12a-12e may be virtual modules. Moreover, modules 12a-12e may be integrated to form one single real or virtual unit or can be embodied by computer code segments configured to execute the corresponding method steps running on computing unit 12.

Interface unit 11 may be configured to allow remote platform 10 to receive information from and/or transmit information to other devices in system 1 via a network. In particular, interface unit 11 may be configured to interface remote platform 10 with mobile user devices 100 and/or data base 300. The network may include one or more wired and/or wireless networks. For example, the network may include a cellular network (e.g., a long-term evolution (LTE) network, a code division multiple access (CDMA) network, a 3G network, a 4G network, a 5G network, another type of next generation network, etc.), a public land mobile network (PLMN), a local area network (LAN), a wide area network (WAN), a telephone network (e.g., the Public Switched Telephone Network (PSTN)), a private network, an ad hoc network, an intranet, the Internet, a fiber optic-based network, a cloud computing network, or the like, and/or a combination of these or other types of networks.

User interface 13 may comprise a physical interface allowing a user U of remote platform 10 to review monitoring data MD and/or predictive disease states PDS of one or more patients P. User interface 13 may be embodied by a computer workstation comprising a display and input devices such as a keyboard, a mouse, a microphone and so forth. In some implementations, user interface 13 may be configured to generate graphical output for monitoring data MD and/or healthcare data HD and/or for an analysis result as provided by computing unit 12. According to some implementations, user interface 13 may be part of interface unit 11.

Memory unit 14 may comprise one or more real or virtualized storages. Memory unit 14 may be realized as a cloud storage. Alternatively, memory unit 14 may be realized as a local or spread storage within the premises of remote platform 10. Memory unit 14 may comprise one or more memory devices. A plurality of repositories or data bases may be configured in memory unit 14. One may be a model repository configured to store or archive a plurality of prediction models. Further, data base 300 may be part of memory unit 14. According to other implementations, data base 300 may be part of a healthcare information system. The healthcare information system may be a local system within the premises of a healthcare organization. Moreover, the healthcare information system may be cloud based. The healthcare information system may comprise one or more standardized components such as a picture archiving and communication system (PACS), a radiology information system (RIS), a laboratory information system (LIS), a hospital information system (HIS), or an electronic medical record system (EMR). For communication with the healthcare information system in order to access date base 300, interface unit 11 may be configured according to an appropriate standard compatible with the respective healthcare information system, such as the HL7 FHIR standard.

It is to be noted that the number and arrangement of devices and/or components shown in Figures 1 to 3 are provided as an example. In practice, system 1 may include additional devices and/or components, fewer devices and/or components, different devices and/or components, or differently arranged devices and/or components than those shown in Figures 1 to 3. Specifically, there may be additional and/or different medical information devices 200 connected to each mobile user device 100. Moreover, also individual user devices 100 themselves may comprise built-in sensors with which monitoring data MD can be collected. What is more, the number of mobile user devices 100 is not limited to the number of mobile user devices 100 shown. In practice, any number of mobile user devices 100 may be in data communication with remote platform 10. For instance, more than 100, more than 1000 or even more than 10000 mobile user devices 100 may be interfaced with remote platform 10.

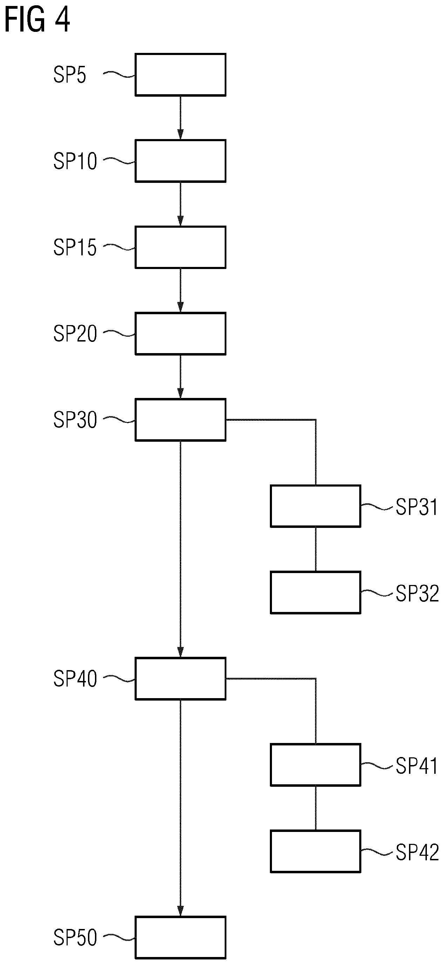

**Figure 4** depicts an inventive method for determining a predictive disease status PDS of a patient P suffering from an inflammatory disease. The order of the steps does not necessarily correspond to the numbering of the steps but may also vary between different embodiments of the present invention. Further, individual steps or sequences of steps may be repeated. The steps shown in Figure 4 happen at remote platform 10 and/or are executed by remote platform 10.

At step SP10, monitoring data MD of patient P is received from mobile user device 100. According to some examples, step SP10 may be preceded by optional step SP5 comprising actively requesting monitoring data MD from mobile user device 100 by remote platform 10. For instance, this may be initiated by manually selecting patient P in a worklist by a user U of remote platform 10. According to some implementations, data transmission of monitoring data MD from mobile user device 100 to remote platform 10 may be (automatically) requested in regular intervals (e.g., once or twice a day) or upon the occurrence of certain events, such as a potential medical issue with a further patient that has a similar medical disposition as patient P (see below). Step SP10 (and step SP5) is optional, as predictive disease status may also be determined based on healthcare data HD, only.

At step SP20, healthcare data HD of patient P is received from data base 300. Like with step SP10, step SP20 may be preceded by an optional step SP15 which comprises actively requesting healthcare data HD from data base 300. Further, this may involve querying data base 300 for healthcare data HD for patient P. Step SP20 (and step SP15) is optional, as predictive disease status may also be determined based on monitoring data MD, only. Step SP20 may further comprise extracting one or more healthcare attributes from healthcare data HD.

At step SP30, a predictive disease status PDS for patient P is determined based on monitoring data MD and/or healthcare data HD. According to some implementations, predictive disease status PDS may relate to the occurrence of a flare of patient P and comprise a flare probability. The determination of predictive disease state PDS at step SP30 may be rulebased. For instance, a number of values and/or parameters may be extracted from monitoring data MD and/or healthcare data HD. The extracted values and/or parameters may then be compared to predetermined thresholds or disease patterns to infer predictive disease status PDS. According to other implementations, a prediction model may be employed which is configured to provide predictive disease status PDS based on monitoring data MD and/or healthcare data HD.

According to some examples, remote platform 10 may host a plurality of different prediction models specialized for different patient cohorts, diseases, disease states, treatment options, medications and so forth. In that case, step SP30 may comprise optional step SP31 directed to automatically select one prediction model from the available prediction models for the current use case based on monitoring data MD and/or healthcare data HD.

Further, step SP30 may comprise optional step SP32 directed to also provide an indication of how much individual parameters comprised in monitoring data MD and/or healthcare data HD affect predictive disease status PDS. At step SP32 parameters and values may be identified which particularly contribute to a certain disease status PDS. This may provide patient P and users U of remote platform 10 with an idea which of the influencing factors comprised in monitoring data MD and/or healthcare data HD are pertinent and where counter measures are particularly promising. Step SP32 may be denoted as "explainable computation" step.

At step SP40, predictive disease status PDS is evaluated. This may involve comparing predictive disease status PDS to a threshold. For instance, a flare probability may be compared with a threshold. If the flare probability is below the threshold, no actions will be required whereas further actions will be triggered if the flare probability is above the threshold. According to some examples, step SP40 may comprise optional step SP41 directed to determine a clinical stage of predictive disease status PDS. This may be done by mapping predictive disease status PDS to a clinical guideline for the underlying disease. That followed, in further optional step SP42, a set of actions may be determined based on the stage identified at step SP41. This may again be done based on an appropriate clinical guideline and/or a predetermined association linking stages with appropriate actions. Actions could be, for instance, dispatching an alert to patient P or the responsible user U at remote platform 10 or initiating a call between patient P and user U.

At step SP50, predictive disease status PDS is provided. This may mean communicating predictive disease status PDS to patient P and/or the responsible user U at remote platform 10. Further, this may involve putting those actions into practice which were determined at previous step SP40, for instance. According to some examples, "providing" may also comprise adding predictive disease status PDS to healthcare data HD of patient P. Further, also monitoring data MD may be added to healthcare data HD at step SP50.

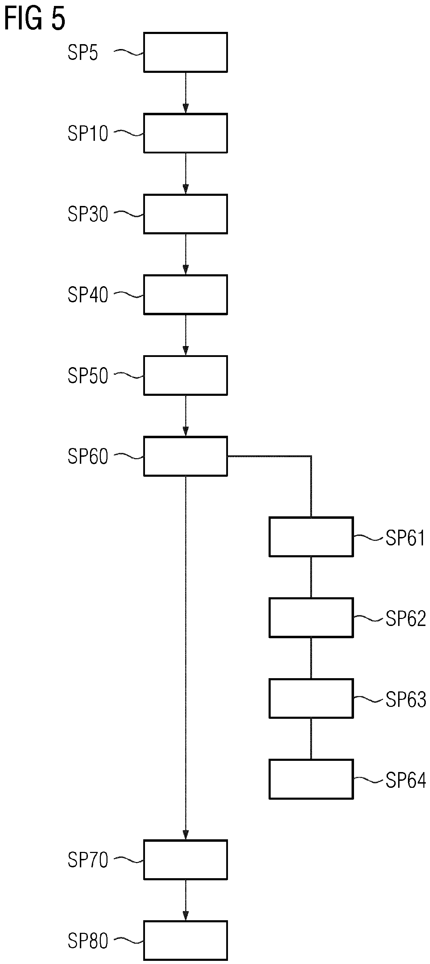

**Figure 5** depicts an alternative method for determining a predictive disease status PDS of a patient P suffering from an inflammatory disease. The order of the steps does not necessarily correspond to the numbering of the steps but may also vary between different embodiments of the present invention. Further, individual steps or sequences of steps may be repeated. The steps shown in Figure 5 happen at remote platform 10 and/or are executed by remote platform 10. Like reference numerals as compared to Figure 4 relate to the same or similar steps.

The main difference between the method of Figure 4 and the method of Figure 5 is that the method of Figure 5 additionally comprises step SP60 directed to identify similar patients with respect to patient P. Step SP60, for instance, may be triggered based on predictive disease status PDS. In particular, step SP60 may be carried out if predictive disease status PDS indicates a potential health issue for patient P such as the likely occurrence of a flare in the near future. To identify similar patients, the following optional sub-steps SP61 to SP64 may be performed.

At sub-step SP61, a data descriptor is generated from monitoring data MD and/or healthcare data HD. The data descriptor may comprise the key features of monitoring data MD and/or healthcare data HD in the form of a feature vector. The feature vector may comprise one or more parameters characterizing the patient P and or the patient's health state relative to the ensemble of patients monitored at remote platform 10. In some examples, the step of generating a data descriptor may be carried out using a trained machine-learning algorithm (also denoted as trained function). Preferably, the trained machine-learning algorithm comprises a neural network, most preferably a convolutional neural network. A first group of neural network layers may be applied to extract features from data. The thus extracted features may be fed as input values to a second group of network layers, also known as classifiers, which serve to further assign objects and/or characteristics to at least one of the extracted features. According to other implementations, the machine-learning algorithm may also comprise a Random Forest algorithm.

At step SP62, the data descriptor of patient P is compared to corresponding data descriptors of further patients. The data descriptors of the further patients may be generated in the same way as the data descriptor for patient P. Specifically, monitoring data MD and/or healthcare data HD of the further patients may be requested for that purpose, if needed. For easy reference, monitoring data MD and/or healthcare data HD of the further patients may also be denoted as secondary monitoring data MD and/or secondary healthcare data HD.

At step SP63, a similarity metric representing a similarity between the data descriptor of patient P and the data descriptors of at least a part of the further patients is determined. In some examples, the similarity metric may be a distance in vector space between the data descriptor of patient P and the data descriptors of the further patients. For example, the distance may be the Euclidean distance between the two points in vector space that the respective data descriptors represent. In some examples, the similarity metric may be the L1 norm of the respective data descriptors. In some examples, other similarity metrics may be used, such as a cosine similarity between the data descriptors. For each further patient taken into account, the similarity metric may represent how similar the reference case is to the target patient. In other words, the similarity metric expresses (quantifies) a degree of similarity between the target patient and a respective reference patient.

Next, at step SP64, the degrees of similarity determined in step SP63 may be used to select those further patients having a certain similarity to patient P. According to some examples, all further patients may be selected the degree of similarity to patient P is greater than a predetermined threshold.

Due to the similarity between patient P and the selected similar patients, there is a certain likelihood that predictive disease status PDS may also be pertinent for the determined similar patients. In other words, if predictive disease status PDS indicates a problem for patient P there likely will be similar problems for the similar patients. Therefore, at optional step SP70, predictive disease status PDS may be provided to the similar patients and/or the responsible users U at remote platform 10 associated to the similar patients. Apart from that, step SP70 corresponds to step SP50 as explained previously. In addition to that or as an alternative, the knowledge about the similar patients may be used in optional steps SP80 to initiate a check of the health status of the similar patients. To this end, the steps SP5 to SP50 may be carried out for each of the similar patients.

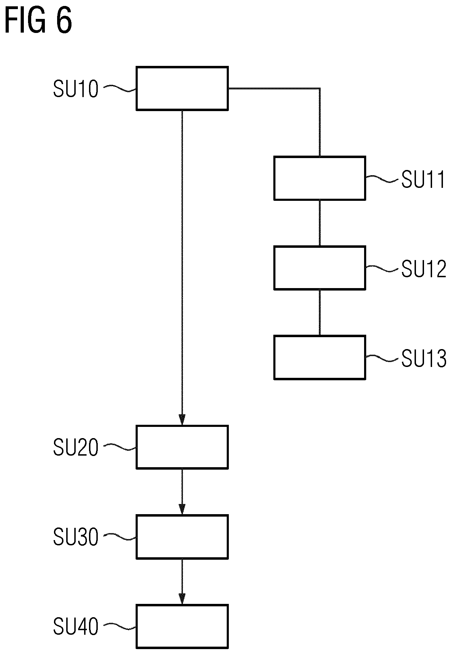

**Figure 6** depicts an inventive method for determining a predictive disease status PDS of a patient P suffering from an inflammatory disease according to another example. The order of the steps does not necessarily correspond to the numbering of the steps but may also vary between different embodiments of the present invention. Further, individual steps or sequences of steps may be repeated. The steps shown in Figure 6 happen at mobile user device 100 and/or are executed by mobile user device 100.

At step SU10, mobile user device 100 collects monitoring data MD. This may comprise optional sub-steps SU11, SU12, SU13. Step SU11 is directed to collect monitoring data MD from medical information devices 200. This may involve prompting medical information devices 200 to measure one or more physiological measurement values of patient P and transmit these to mobile user device 100 essentially as explained before.

Step SU12 is directed to gather monitoring data MD from inputs of patient P into mobile user device 100. For instance, patient P may be provided with an electronic questionnaire in which she or he is supposed to indicate the overall wellbeing, the number of swollen joints, the diet, the medication taken and so forth. Inputs may, for instance, be made by activating checkboxes, clicking on symbols, or filling input fields in an appropriate graphical user interface running at mobile user device 100. Further, inputs may be made by free-text messages or voice commands which may be further processed by computer linguistic algorithms to infer their intended meaning.

Step SU13 is directed to collect monitoring information MD in the form of local environmental information relevant for the patient's health state essentially as described before. In particular, SU13 is directed to collect monitoring data MD from external data sources 400.

At step SU20, mobile user device 100 transmits monitoring data MD to remote platform 10 using the data transmission means as herein described.

At step SU30, mobile user device 100 receives predictive disease status PDS as determined at remote platform 10 for patient P. The message from remote platform 10 forwarding predictive disease status PDS may also comprise instructions for mobile user device 100 and/or patient P which actions are to be taken next. For instance, the message from remote platform 10 may comprise an instruction to display predictive disease status PDS and/or a synopsis of predictive disease status PDS to patient P. Further, the message may comprise instructions to provide patient P with recommendations or instructions for further actions.

At step SU40, predictive disease status PDS is provided to patient P. This may involve bringing predictive disease status PDS to the attention of patient P, optionally, together with any recommendation and/or instruction as received from remote platform 10.

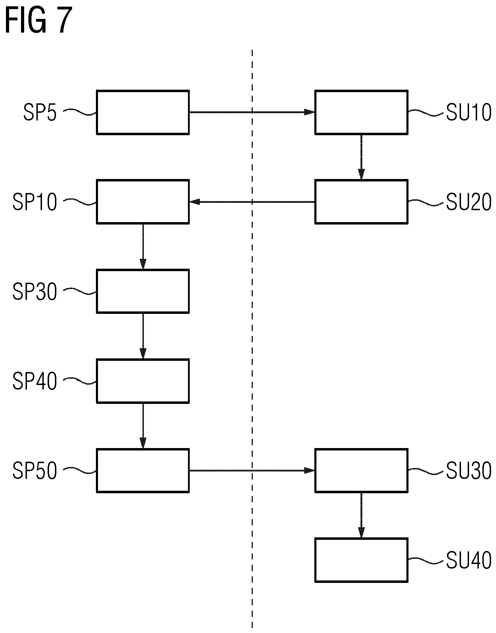

**Figure 7** depicts a basic sequence of method steps for how remote platform 10 and mobile user device 100 may work together to implement a method for determining a predictive disease status PDS of a patient P suffering from an inflammatory disease according to an embodiment. The steps shown on the left-hand side of Figure 7 are executed by remote platform 10 (i.e., remote platform 10 is configured to execute these steps), whereas the steps shown on the right-hand side are executed by mobile user device(s) 100 (i.e., mobile user device(s) 100 are respectively configured to execute these steps). The steps shown in Figure 7 correspond to the steps of Figures 4 to 6. For easy reference, several optional steps explained in connection with Figures 4 to 6 have been omitted but may of course be added if needed to form additional implementations.

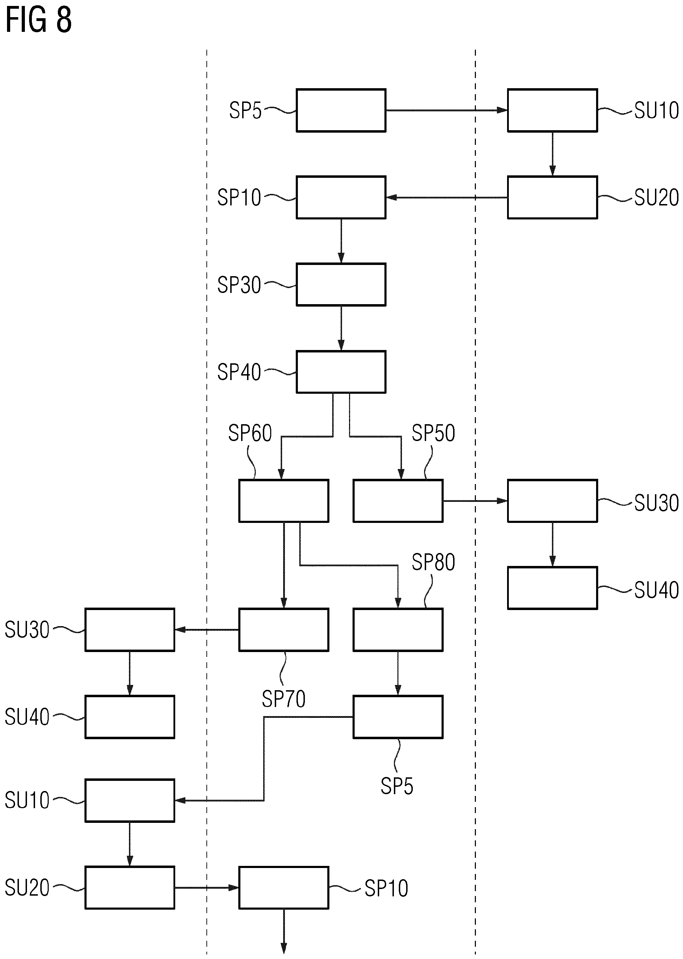

**Figure 8** depicts a basic sequence of method steps for how remote platform 10 and mobile user device 100 may work together to implement a method for determining a predictive disease status PDS of a patient P suffering from an inflammatory disease according to another embodiment. The implementation shown in Figure 8 further illustrates the integration of the similar patient search introduced in connection with Figure 6 into the previous workflows. The steps shown in the middle of Figure 8 are executed by remote platform 10, the steps shown on the right-hand side are executed by mobile user device 100 of patient P, and the steps on the left-hand side of Figure 8 are executed by a secondary mobile user device 100 associated to a patient similar to patient P. Otherwise, the steps shown in Figure 8 correspond to the steps of Figures 4 to 7. For easy reference, several optional steps explained in connection with Figures 4 to 7 have been omitted but may of course be added if needed to form additional implementations.

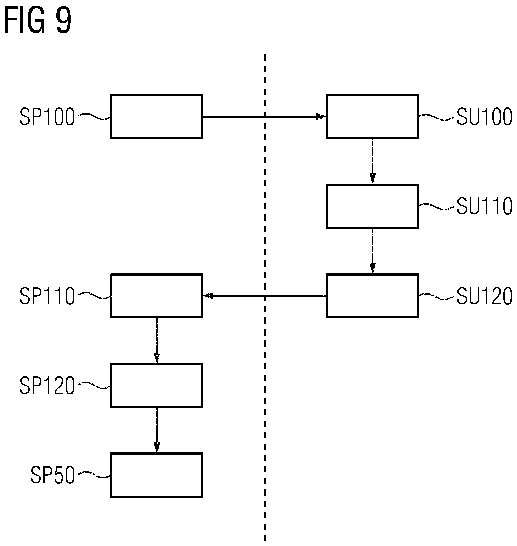

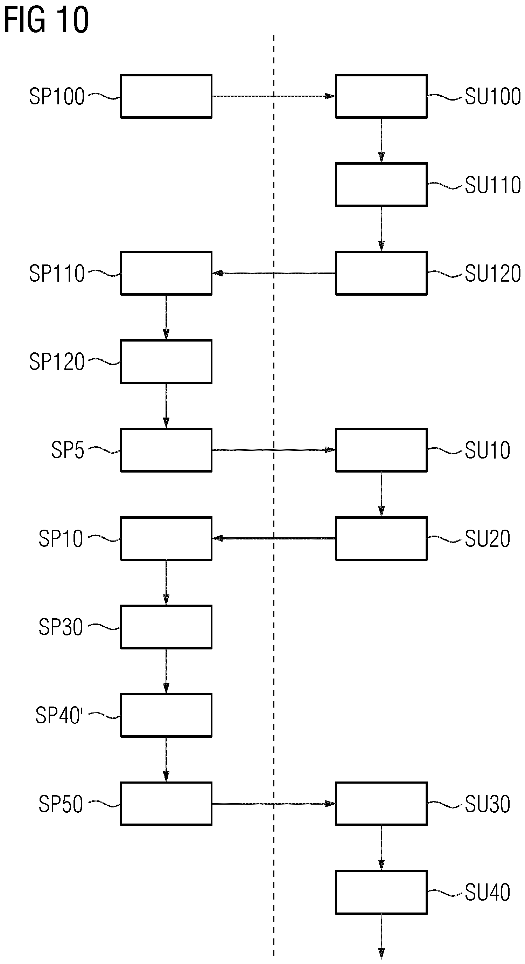

**Figures 9** and **Figure 10** depict a basic sequence of method steps for how remote platform 10 and mobile user device 100 may work together to implement a method for determining a predictive disease status PDS of a patient P suffering from an inflammatory disease according to another embodiment. The implementation shown in Figures 9 and 10 differs from the previous implementations in that mobile user device 100 is provided with local prediction capabilities in the form of a prediction model. The steps shown on the left-hand side of Figures 9 and 10 are executed by remote platform 10, the steps shown on the right-hand side are executed by mobile user devices 100. In the following only those steps will be explained which deviate from the previous embodiments. For the other steps reference is made to the explanations given in connection with Figures 4 to 8. For easy reference, several optional steps of the previous embodiments have been omitted but may of course be added if needed to form additional implementations. In particular, the implementation of Figures 9 and 10 may be combined with the similar patient search by virtue of step SP60 (and, optionally, steps SP70 and/or SP80) .

At step SP100, a prediction model is provided to mobile user device 100 by remote platform 10. That is, the prediction model may be downloaded to mobile user device 100. The prediction model may be fit for local deployment at mobile user device 100 in the sense that it is capable of locally determining a predicative disease status PDS of patient P based on monitoring data MD.

At step SU100, the prediction model is received at mobile user device 100. That followed, at step SU110, the received prediction model may be applied to monitoring data MD in order to locally determine a predictive disease status PDS of patient P. Monitoring data MD may be collected essentially as described in connection with step SU10. To locally determine predictive disease status PDS, computing unit 102 may execute or apply the downloaded prediction model.

At step SU120, the locally determined predictive disease status PDS is provided - either to patient P at mobile user device 100 or to remote platform 10. The latter case may involve data transmission essentially as described before. Together with the locally determined predictive disease status PDS, a log file may be provided indicative of how well the prediction model performed at mobile user device 100. Step SU120 may comprise an optional sub-step of evaluating the locally determined disease status PDS at mobile user device 100 which may be executed essentially as described in connection with step SP40. Dependent on the evaluation it may be decided if and how locally determined predictive disease status PDS is brought to the attention of patient P.

At step SP110, locally determined disease status PDS is received at remote platform 10. That followed, at step SP120, locally determined disease status is evaluated. In essence, step SP120 may be executed as explained previously in connection with step SP40. In addition to that, the optionally transmitted log file may be read in order to determine how accurate the determination result of the locally operating prediction model is.

Dependent on the evaluation at step SP120, locally determined predictive disease status PDS may either be provided to the responsible user U at remote platform 10 via step SP50 (Figure 9) or further checked at remote platform 10 (Figure 10). In the latter case, locally determined predictive disease status PDS may be compared with a predictive disease status PDS being determined at remote platform 10 with a different (better) predictive model and/or additional data, e.g., in the form of healthcare data HD of patient P. To do so, remote platform 10 may execute steps SP5 to SP50 essential as described before - with a possible difference in that a comparison of locally determined predictive disease status PDS with the predictive disease status determined at remote platform 10 may take place at modified step SP40'.

Wherever meaningful, individual embodiments or their individual aspects and features can be combined or exchanged with one another without limiting or widening the scope of the present invention. Advantages which are described with respect to one embodiment of the present invention are, wherever applicable, also advantageous to other embodiments of the present invention.

The following points are also part of the disclosure:
1. Computer-implemented method for predicting a disease status (PDS) of an inflammatory disease for a patient (p), in a system (1) comprising a remote platform (10) and at least one mobile user device (100), the mobile user device (100) being associated to the patient (P) and being in data communication with the platform (10),
   the method comprising the following steps:
   receiving (SP10), at the remote platform (10), monitoring data (MD) indicative of the health state of the patient (P) from the mobile user device (100) associated to the patient (P),
   determining (SP30), at the remote platform (10), a predictive disease status (PDS) of the inflammatory disease of the patient (P) based on the received monitoring data (MD);
   evaluating (SP40), at the remote platform (10), the determined predictive disease status (PDS); and
   providing (SP50) the predictive disease status (PDS) to a user (U) at the remote platform (10) and/or to the mobile user device (100) based on the step of evaluating (SP40) the determined predictive disease status (PDS).
2. Method according to 1, wherein the predictive disease status (PDS) relates to at least one of:
   a predictive disease activity of the inflammatory disease of the patient (P), in particular, to an occurrence of a flare and/or exacerbation of the inflammatory disease of the patient (P), and/or
   a predictive treatment response of the patient (P) with respect to a treatment of the inflammatory disease of the patient (P), and/or
   a predictive occurrence of an adverse effect related to a treatment of the inflammatory disease of the patient (P).
3. Method according to any one of the preceding points, wherein:
   the remote platform (10) is in data communication with a local or cloud-based data base (300) for storing electronic medical records of patients,
   the method further comprises a step of retrieving (SP20), by the remote platform (10), healthcare data (HD) associated to the patient (P) from the at least one data base (300); and
   the step of determining (SP30) the predictive disease status is additionally based on the healthcare data (HD).
4. Method according to 3, further comprising:
   adding the received monitoring data (MD) to the retrieved healthcare data (HD) of the patient and/or storing the received monitoring data (MD) of the patient (P) in the data base (300) in association with the healthcare data (HD) of the patient (P).
6. Method according to any one of the preceding points, wherein:
   the mobile user device (100) is configured to receive an input of the patient (P) indicative of a health state information of the patient (P) as perceived by the patient (P), the health state information comprising one or more answers of the patient (P) to a questionnaire; and
   the monitoring data (MD) comprises the health state information.
7. Method according to 6, further comprising:
   configuring the questionnaire based on the healthcare data (HD), the monitoring data (MD) and/or the predictive disease status (PDS); and
   forwarding the questionnaire to the mobile user device (100) for providing the questionnaire to the patient (P).
8. Method according to 6, further comprising:
   selecting the questionnaire from a plurality of preconfigured questionnaires based on the healthcare data (HD), the monitoring data (MD) and/or the predictive disease status (PDS); and
   forwarding the selected questionnaire to the mobile user device (100) for providing the questionnaire to the patient (P) ;
   the preconfigured questionnaires preferably comprising at least one of a Health Assessment Questionnaire (HAQ), a Visual Analog Scale (VAS) questionnaire, and/or a Rheumatoid Arthritis Impact of Disease (RAID) questionnaire
9. Method according to any one of the preceding points, further comprising the steps:
   establishing a bi-directional communication channel between the remote platform (10) and the mobile user device (100) based on the step of evaluating (SP40) the determined predictive disease status (PDS), the communication channel enabling a direct communication between patient and platform user.
9. Method according to any one of the preceding points, wherein:
   the remote platform (10) is configured to host a plurality of different prediction models each configured and/or trained to determine a predictive disease status (PDS) of an inflammatory disease of a patient (P) based on input data; and
   the method further comprises a step of selecting (SP31) one of the hosted prediction models depending on at least the received monitoring data (MD); and
   the step of determining (SP30) the predictive disease status (PDS) comprises applying the selected prediction model to at least the received monitoring data (MD).
10. Method according to any of the preceding points, further comprising the steps:
   deriving (SP32) values of how much individual parameters comprised in the monitoring data (MD) affect the predictive disease status (PDS); and
   providing (SP50) at least one of the derived values and/or the corresponding parameters to a user (U) at the remote platform (10) and/or to the mobile user device (100) in conjunction with the determined predictive disease status (PDS).
11. Method according to any of the preceding points, wherein:
   the remote platform (10) is in wireless data communication with a plurality of secondary mobile user devices (100) each secondary mobile user device (100) being respectively associated to a further patient different from the patient (P); and
   the method further comprises the steps:
      searching (SP60) the further patients for similar patients, the similar patients having a degree of similarity to the patient (P) for which the predictive disease status has been determined (SP30) and evaluated (SP40); and
      providing (SP70) the predictive disease status (PDS) to a user (U) at the remote platform (10) in connection with the similar patients and/or to the mobile user device (100) of the similar patients; and/or
      respectively determining (SP30) a secondary predictive disease status for each of the similar patients and comparing (SP80) each of the secondary predictive disease states with the predictive disease status (PDS).
12. Method according to 11, wherein:
   the method further comprises at least one of the following two steps:
      receiving (SP10), at the remote platform (10), secondary monitoring data (MD) indicative of the health states of the further patients from the secondary mobile user devices (100), and/or
      retrieving (SP20) healthcare data (HD) associated to the patient (P) and secondary healthcare data (HD) associated to the further patients from a data base (300) for storing electronic medical records of patients, wherein the remote platform (10) is in data communication with the data base (300); and
   the step of searching (SP60) is based on a comparison of the secondary healthcare data (HD) with the healthcare data (HD) and/or a comparison of the secondary monitoring data (MD) with the monitoring data (MD).
13. Computer-implemented method for predicting a disease status (PDS) for an inflammatory disease of a patient (P), in a system (1) comprising a remote platform (10) and at least one mobile user device (100), the mobile user device (100) being associated to the patient (P) and being in wireless data communication with the remote platform (10),
   the method comprising the following steps:
   collecting (SU10), at the mobile user device (100), monitoring data (MD) indicative of the health state of the patient (P),
   transmitting (SU20) the monitoring data (MD) to the remote platform (10), in order to determine, at the remote platform (10) predictive disease status (PDS) of the inflammatory disease of the patient (P) based on the transmitted monitoring data (MD);
   receiving (SU30), at the mobile user device (100), from the remote platform (10), information indicative of the determined predictive disease status (PDS); and
   providing (SU40), to the patient (P), an output indicative of the received information.
14. Method according to 13, further with the steps:
   receiving (SU100), at the mobile user device (100), a prediction model configured and/or trained to locally determine, at the mobile user device (100), a predictive disease status (PDS) of the inflammatory disease of the patient (P) based on the monitoring data (MD);
   locally determine (SU110), at the mobile user device (100) a predictive disease status (PDS) for the patient (P) by applying the prediction model to the monitoring data (MD); and
   providing (SU120) an information indicative of the locally determined predictive disease status (PDS) to the patient (P) at the mobile user device (100) and/or to the remote platform (10).
15. Method according to 14, further comprising:
   further training one or more prediction models at the remote platform (10) and/or the mobile user device (100) using the information indicative of the locally determined predictive disease status (PDS) and, optionally, the monitoring data (MD) and/or the healthcare data (HD).

## Claims

1. Computer-implemented method for predicting a disease status (PDS) of an inflammatory disease for a patient (p), in a system (1) comprising a remote platform (10) and at least one mobile user device (100), the mobile user device (100) being associated to the patient (P) and being in data communication with the platform (10),
the method comprising the following steps:
receiving (SP10), at the remote platform (10), monitoring data (MD) indicative of the health state of the patient (P) from the mobile user device (100) associated to the patient (P),
determining (SP30), at the remote platform (10), a predictive disease status (PDS) of the inflammatory disease of the patient (P) based on the received monitoring data (MD);
evaluating (SP40), at the remote platform (10), the determined predictive disease status (PDS); and
providing (SP50) the predictive disease status (PDS) to a user (U) at the remote platform (10) and/or to the mobile user device (100) based on the step of evaluating (SP40) the determined predictive disease status (PDS).

2. Method according to claim 1, wherein the predictive disease status (PDS) relates to at least one of:
a predictive disease activity of the inflammatory disease of the patient (P), in particular, to an occurrence of a flare and/or exacerbation of the inflammatory disease of the patient (P), and/or
a predictive treatment response of the patient (P) with respect to a treatment of the inflammatory disease of the patient (P), and/or
a predictive occurrence of an adverse effect related to a treatment of the inflammatory disease of the patient (P).

3. Method according to any one of the preceding claims, wherein:
the remote platform (10) is in data communication with a local or cloud-based data base (300) for storing electronic medical records of patients,
the method further comprises a step of retrieving (SP20), by the remote platform (10), healthcare data (HD) associated to the patient (P) from the at least one data base (300); and
the step of determining (SP30) the predictive disease status is additionally based on the healthcare data (HD).

4. Method according to claim 3, wherein in the healthcare data (HD) comprises at least one of: information about a medication prescribed to treat the inflammatory disease of the patient (P), information about a medication dose prescribed to treat the inflammatory disease of the patient (P), demographic information of the patient (P), medical image data of the patient (P), laboratory data of the patient (P), prior monitoring data acquired from the patient (P), information concerning the patient's (P) lifestyle, information about the disease history of the patient (P), and/or information about previous examinations of the patient (P).

5. Method according to any one of the preceding claims, wherein:
the mobile user device (100) is in data connection with at least one medical information device (200) configured to determine one or more physiological measurement values of the patient (p), the medical information device (200) preferably being selected from one of a weight scale, a pulse oximeter, a blood pressure meter, a heart rate monitor, an activity tracker, a thermometer, an airflow sensor, a galvanic skin response sensor, and/or a blood glucose meter; and
the monitoring data (MD) comprises one or more physiological measurement values as determined by the at least one medical information device (200).

6. Method according to any one of the preceding claims, wherein:
the mobile user device (100) is configured to receive an input of the patient (P) indicative of a health state information of the patient (P) as perceived by the patient (P), the health state information preferably comprising at least one of: an indication of the patient's (P) perceived wellbeing, a number of swollen and/or tender joints as determined by the patient (P), a patient's (P) diet, a medication having been taken by the patient (P), an occurrence or beginning of a flare as perceived by the patient (P), one or more answers of the patient (P) to a questionnaire, and/or an adverse effect related to the medication prescribed to the patient (P) as perceived by the patient (P); and
the monitoring data (MD) comprises the health state information.

7. Method according to any one of the preceding claims, wherein:
the mobile user device (100) is configured to gather current and/or prospective local environmental information apt to influence the patient's (P) health state, the local environmental information preferably comprising at least one of: current weather conditions, current air pollution values, current allergen concentrations, prospective weather conditions, prospective air pollution values, and/or prospective allergen concentrations; and
the monitoring data (MD) comprises the local environmental information.

8. Method according to any one of the preceding claims, wherein:
the remote platform (10) is configured to host at least one prediction model configured and/or trained to determine a predictive disease status (PDS) of an inflammatory disease of a patient (P) based on input data; and
the step of determining (SP30) the predictive disease status (PDS) comprises applying the at least one prediction model to at least the received monitoring data (MD).

9. Method according to any one of the preceding claims, wherein:
the remote platform (10) is in wireless data communication with a plurality of secondary mobile user devices (100) each secondary mobile user device (100) being respectively associated to a further patient different from the patient (P); and
the method further comprises the steps:
searching (SP60) the further patients for similar patients, the similar patients having a degree of similarity to the patient (P) for which the predictive disease status has been determined (SP30) and evaluated (SP40); and
providing (SP70) the predictive disease status (PDS) to a user (U) at the remote platform (10) in connection with the similar patients and/or to the mobile user device (100) of the similar patients; and/or
respectively determining (SP30) a secondary predictive disease status for each of the similar patients and comparing (SP80) each of the secondary predictive disease states with the predictive disease status (PDS).

10. Method according to any one of the preceding claims, wherein:
the method further comprises a step of providing a predetermined association, the predetermined association linking different stages of predictive disease states (PDS) to actions and/or recommendations for the patient (P) and/or a user (U) of the remote platform (10);
the step of evaluating (SP40) comprises determining a stage of the determined predictive disease status (PDS) and selecting actions and/or recommendations based on the determined stage and the predetermined association; and
providing (SP50) selected actions and/or recommendations to a user (U) at the remote platform (10) and/or to the mobile user device (100).

11. Method according to any one of the preceding claims, further with the steps:
providing (SP100), to the mobile user device (100), a prediction model configured and/or trained to locally determine, at the mobile user device (100), a predictive disease status (PDS) of the inflammatory disease of the patient (P) based on the monitoring data (MD);
receiving (SP110), at the remote platform (10), a locally determined predictive disease status (PDS); and, optionally,
comparing (SP120), at the remote platform (10), the locally determined predictive disease status (PDS) for the patient (P) with the determined predictive disease status (PDS) as determined at the remote platform (10).

12. Patient monitoring platform (10) for predicting a disease status (PDS) for an inflammatory disease of a patient (P), the platform (10) comprising:
an interface unit (11) configured to communicate with at least one mobile user device (100) being associated to the patient (P) and arranged at a remote location from the platform (10) for receiving monitoring data (MD) indicative of the health state of the patient (P) from the mobile user device (100); and
a computing unit (12) configured to:
determine (SP30) a predictive disease status (PDS) for the patient (P) based on the received monitoring data (MD) ;
evaluate (SP40) the determined predictive disease status (PDS); and
provide (SP50), based on the evaluation, the predictive disease status (PDS) to a user (U) at the remote platform (10) and/or the mobile user device (100).

13. System (1) for predicting a disease status (PDS) for an inflammatory disease of a patient (P), comprising a platform (10) according to claim 12 and at least one mobile user device (100) associated to the patient (P), the mobile user device (100) being configured to collect monitoring data (MD) indicative of the health state of the patient (P) and to transmit the monitoring data (P) to the platform (10).

14. Computer program product comprising program elements which induce a computing unit (12, 102) of a system for predicting a disease status (PDS) for an inflammatory disease of a patient (P) to perform the steps according to the method of any of claims 1 to 11, when the program elements are loaded into a memory of the computing unit (12, 102).

15. Computer-readable medium on which program elements are stored that are readable and executable by a computing unit (12, 102) of a system for predicting a disease status (PDS) for an inflammatory disease of a patient (P), in order to perform steps of the method according to any of claims 1 to 11, when the program elements are executed by the computing unit (12, 102).
